# EUROPEAN PATENT SPECIFICATION

(11) **EP 1 945 819 B1**
(45) Date of publication and mention of the grant of the patent: **20.03.2013**
(21) Application number: 06837364.6
(22) Date of filing: 10.11.2006
(51) Int. Cl.: A61K 48/00, A61K 39/395, A61K 38/17, C12Q 1/68, G01N 33/50, G01N 33/574

(54) **GENE EXPRESSION PROFILES AND METHODS OF USE**
GENEXPRESSIONSPROFILE UND ANWENDUNGSVERFAHREN
PROFILS D'EXPRESSION GENIQUE ET PROCEDES D'UTILISATION

(30) Priority: 12.11.2005 US 735581 P
(43) Date of publication of application: 23.07.2008
(73) Proprietor: Siemens Healthcare Diagnostics Inc., Tarrytown, NY 10591 (US)
(72) Inventor: TAYLOR, Ian, Madison, CT 06443 (US); BIGWOOD, Douglas, Madison, CT 06443 (US)
(74) Representative: Maier, Daniel Oliver
(86) International application number: PCT/US2006/043855
(87) International publication number: WO 2007/058968

(56) References cited:
- WO-A-2005/000098
- "GENECHIP HUMAN GENOME ARRAYS - THE MOST COMPREHENSIVE COVERAGE OF ALL WELL-SUBSTANTIATED GENES IN THE HUMAN GENOME" INTERNET CITATION, [Online] 2004, XP002449668 Retrieved from the Internet: URL:http://www.affymetrix.com/support/tech nical/datasheets/human_datasheet.p> [retrieved on 2007-01-01]
- ABOU-ALFA GHASSAN K ET AL: "Phase II study of sorafenib in patients with advanced hepatocellular carcinoma." JOURNAL OF CLINICAL ONCOLOGY : OFFICIAL JOURNAL OF THE AMERICAN SOCIETY OF CLINICAL ONCOLOGY 10 SEP 2006, vol. 24, no. 26, 10 September 2006 (2006-09-10), pages 4293-4300, XP002540848 ISSN: 1527-7755
- STRUMBERG DIRK ET AL: "Raf kinase inhibitors in oncology" ONKOLOGIE, KARGER, FREIBURG, DE, vol. 28, no. 2, 1 January 2005 (2005-01-01), pages 101-107, XP009104989 ISSN: 0378-584X
- STRUMBERG DIRK ET AL: "Phase I clinical and pharmacokinetic study of the Novel Raf kinase and vascular endothelial growth factor receptor inhibitor BAY 43-9006 in patients with advanced refractory solid tumors" JOURNAL OF CLINICAL ONCOLOGY, AMERICAN SOCIETY OF CLINICAL ONCOLOGY, US, vol. 23, no. 5, 10 February 2005 (2005-02-10), pages 965-972, XP002420707 ISSN: 0732-183X
- POSADAS E.M. ET AL.: 'A phase II study of BAY 43-9006 in patients with androgen-independent prostate cancer (AIPC) with proteomic profiling' JOURNAL OF CLINICAL ONCOLOGY vol. 23, no. 16, SUPPL., 01 June 2005, page 4762, XP003024745

## Description

### FIELD OF THE INVENTION

The present invention relates to gene expression profiles, microarrays comprising nucleic acid sequences representing gene expression profiles, and methods of using gene expression profiles and microarrays.

### BACKGROUND OF THE INVENTION

Many disease states are characterized by differences in the expression levels of various genes either through changes in the copy number of the genetic DNA or through changes in levels of transcription of particular genes (e.g., through control of initiation, provision of RNA precursors, RNA processing, etc.). For example, losses and gains of genetic material play an important role in malignant transformation and progression. These gains and losses are thought to be "driven" by at least two kinds of genes, oncogenes and tumor suppressor genes. Oncogenes are positive regulators of tumorgenesis, while tumor suppressor genes are negative regulators of tumorgenesis (Marshall, Cell 64:313-326, 1991; Weinberg, Science 254:1138-1146, 1991). Therefore, one mechanism of activating unregulated growth is to increase the number of genes coding for oncogene proteins or to increase the level of expression of these oncogenes (e.g., in response to cellular or environmental changes), and another mechanism is to lose genetic material or to decrease the level of expression of genes that code for tumor suppressors. This model is supported by the losses and gains of genetic material associated with glioma progression (Mikkelson, et al., J. Cellular Biochem. 46:3-8, 1991). Thus, changes in the expression (transcription) levels of particular genes (e.g., oncogenes or tumor suppressors) serve as signposts for the presence and progression of various cancers.

Compounds which are used as therapeutics to treat these various diseases (e.g., cancer) presumably reverse some, or all, of these gene expression changes. The expression change of at least some of these genes may therefore, be used as a method to monitor, or even predict, the efficacy of such therapeutics. The analysis of these expression changes may be performed in the target tissue of interest (e.g., tumor) or in some surrogate cell population (e.g., peripheral blood leukocytes). In the latter case, correlation of the gene expression changes with efficacy (e.g., tumor shrinkage or non-growth) must be especially strong for the expression change pattern to be used as a marker for efficacy.

A number of laboratories have reported success in using gene expression analysis, via microarrays or other methods, to classify human tumors at the molecular level (Bittner et et al., Nature 403:503-511, 2000; Golub et al., Science 286:531-537, 1999; Perou et al., Proc. Natl. Acad. Sci. 96:9212-9217, 1999; Kahn et al., American Journal of Pathology 156:1887-1900, 2000). Genes, either individually or as a subset, identified in this way could be used as markers that could be tracked for changes that correlate with efficacy of a therapeutic compound(s) or to predict which patients might benefit from a particular therapeutic. Similarly, laboratories have used gene expression profiling of RNA isolated from frozen whole blood or peripheral blood mononuclear cells (PBMCs) to identify gene expression patterns that are disease-associated (e.g. cancer) or that predict sensitivity to a therapy (DePrimo et al, BMC Cancer 3:3, 2003; Kaneta et al, Jpn. J. Cancer Res. 93:849-856, 2002; Hofmann et al, The Lancet 359:481-486, 2002; Whitney et al, Proc. Natl. Acad. Sci. 100:1896-1901, 2003.). In this respect, the blood cells can be thought of as a surrogate system, or circulating biosensor, for the identification of a disease state or a response to a therapy.

Total RNA was isolated from whole blood samples taken from patients with hepatocellular carcinoma enrolled in a clinical trial for sorafenib. The RNA was analyzed from each sample by Affymetrix technology and compared to the patients' response to sorafenib as defined by RECIST or WHO criteria to identify subsets of genes that can be used to predict patient response.

### SUMMARY OF THE INVENTION

The present invention is directed to the method of claim 1 and further discloses gene expression profiles, microarrays comprising nucleic acid sequences representing said gene expression profiles, and methods of using said gene expression profiles and microarrays.

It is further disclosed that the gene expression profile is an expression profile comprising one or more genes (e.g., SEQ ID NOs: 1-18) that demonstrate altered expression following exposure to a drug. For example, the expression profile comprises one or more genes that demonstrate altered expression following exposure to a multi-kinase inhibitor (e.g., sorafenib).

It is further disclosed that the expression profile is an expression profile comprising one or more polypeptides encoding genes (e.g., SEQ ID NOs: 1-18) that demonstrate altered expression following exposure to a drug. For example, the expression profile comprises one or more polypeptides that demonstrate altered expression following exposure to a multi-kinase inhibitor (e.g., sorafenib).

Herein further disclosed are microarrays comprising one or more genes that demonstrate altered expression following exposure to a multi-kinase inhibitor (e.g., sorafenib). In another embodiment of the present invention, the microarray may be a microarray comprising one or more genes selected from the group consisting of the genes listed in Table 1 (SEQ ID NO: 1-18). In a further embodiment, the microarray may be a microarray comprising one or more biomarkers isolated from the group comprising the genes listed in Table 1 (SEQ ID NO: 1-18).

Herein further disclosed is to provide methods and reagents for the prediction, diagnosis, prognosis, and therapy of cancer.

Herein further disclosed is methods for using said microarrays which include, but are not limited to, screening the effects of a drug or treatment (e.g., a multi-kinase inhibitor) on tissue or cell samples, screening toxicity effects on tissue or cell samples, identifying a disease state in a tissue or cell sample, providing a patient diagnosis, predicting a patient's response to treatment, distinguishing between control and drug-treated samples, distinguishing between normal and tumor samples, discovering novel drugs, and determining the level of gene expression in a tissue or cell sample.

Herein further disclosed is a method for screening the effects of a drug (e.g., a multi-kinase inhibitor) on a tissue or cell sample comprising the step of analyzing the level of expression of one or more genes (e.g., SEQ ID NOs: 1-18) and/or gene products, wherein the gene expression and/or gene product levels in the tissue or cell sample are analyzed before and after exposure to the drug, and a variation in the expression level of the gene and/or gene product is indicative of a drug effect or provides a patient diagnosis or predicts a patient's response to the treatment.

Herein further disclosed is a method for discovering novel drugs comprising the step of analyzing the level of expression of one or more genes and/or gene products, wherein the gene expression and/or gene product levels of the cells are analyzed before and after exposure to the drug, and a variation in the expression level of the gene and/or gene product is indicative of drug efficacy.

Herein further disclosed is a method for identifying a compound useful for the treatment of cancer comprising administering to a subject with cancer a test compound, and measuring the activity of the polypeptide, wherein a change in the activity of the polypeptide is indicative of the test compound being useful for the treatment of cancer.

Herein further disclosed are methods which may be used to identify compounds which may act, for example, as regulators or modulators such as agonists and antagonists, partial agonists, inverse agonists, activators, co-activators, and inhibitors. Accordingly, the invention provides reagents and methods for regulating the expression of a polynucleotide or a polypeptide associated with cancer. Reagents that modulate the expression, stability, or amount of a polynucleotide or the activity of the polypeptide may be a protein, a peptide, a peptidomimetic, a nucleic acid, a nucleic acid analogue (e.g., peptide nucleic acid, locked nucleic acid), or a small molecule.

Herein further disclosed is a method for providing a patient diagnosis comprising the step of analyzing the level of expression of one or more genes and/or gene products, wherein the gene expression and/or gene product levels of normal and patient samples are analyzed, and a variation in the expression level of the gene and/or gene product in the patient sample is diagnostic of a disease. The patient samples include, but are not limited to, blood, amniotic fluid, plasma, semen, bone marrow, and tissue biopsy.

Herein further disclosed is a method of diagnosing cancer in a subject comprising measuring the activity of the polypeptide in a subject suspected of having cancer, wherein if there is a difference in the activity of the polypeptide, relative to the activity of the polypeptide in a subject not suspected of having cancer, then the subject is diagnosed has having cancer.

Herein further disclosed is a method for detecting cancer in a patient sample in which an antibody to a protein is used to react with proteins in the patient sample.

Herein further disclosed is a method for distinguishing between normal and disease states comprising the step of analyzing the level of expression of one or more genes and/or gene products, wherein the gene expression and/or gene product levels of normal and disease tissues are analyzed, and a variation in the expression level of the gene and/or gene product is indicative of a disease state.

Herein further disclosed is a method of determining the phenotype of cells comprising detecting the differential expression, relative to normal cells, of at least one gene, wherein the gene is differentially expressed as compared to normal cells.

Herein further disclosed is a method of determining the phenotype of cells, comprising detecting the differential expression, relative to normal cells, of at least one polypeptide, wherein the protein is differentially expressed as compared to normal cells.

Herein further disclosed is a method for determining the phenotype of cells from a patient by providing a nucleic acid probe comprising a nucleotide sequence having at least about 10, at least about 15, at least about 25, or at least about 40 consecutive nucleotides, obtaining a sample of cells from a patient, optionally providing a second sample of cells substantially all of which are non-cancerous, contacting the nucleic acid probe under stringent conditions with mRNA of each of said first and second cell samples, and comparing (a) the amount of hybridization of the probe with mRNA of the first cell sample, with (b) the amount of hybridization of the probe with mRNA of the second cell sample, wherein a difference in the amount of hybridization with the mRNA of the first cell sample as compared to the amount of hybridization with the mRNA of the second cell sample is indicative of the phenotype of cells in the first cell sample.

Herein further disclosed is a test kit for identifying the presence of cancerous cells or tissues, comprising a probe/primer, for measuring a level of a nucleic acid in a sample of cells isolated from a patient. In certain embodiments, the kit may further include instructions for using the kit, solutions for suspending or fixing the cells, detectable tags or labels, solutions for rendering a nucleic acid susceptible to hybridization, solutions for lysing cells, or solutions for the purification of nucleic acids.

Herein further disclosed is a test kit for identifying the presence of cancer cells or tissues, comprising an antibody specific for a protein. In certain embodiments, the kit further includes instructions for using the kit. In certain embodiments, the kit may further include solutions for suspending or fixing the cells, detectable tags or labels, solutions for rendering a polypeptide susceptible to the binding of an antibody, solutions for lysing cells, or solutions for the purification of polypeptides.

Herein further disclosed is a test kit for monitoring the efficacy of a compound or therapeutic in cancerous cells or tissues, comprising a probe/primer, for measuring a level of a nucleic acid in a sample of cells isolated from a patient. In certain embodiments, the kit may further include instructions for using the kit, solutions for suspending or fixing the cells, detectable tags or labels, solutions for rendering a nucleic acid susceptible to hybridization, solutions for lysing cells, or solutions for the purification of nucleic acids.

Herein further disclosed is a test kit for monitoring the efficacy of a compound or therapeutic in cancer cells or tissues, comprising an antibody specific for a protein. In certain embodiments, the kit further includes instructions for using the kit. In certain embodiments, the kit may further include solutions for suspending or fixing the cells, detectable tags or labels, solutions for rendering a polypeptide susceptible to the binding of an antibody, solutions for lysing cells, or solutions for the purification of polypeptides.

Herein further disclosed are methods of identifying biomarkers comprising the steps of selecting a set of biomarker genes from a gene expression profile representing a disease or drug treatment.

### DETAILED DESCRIPTION OF THE INVENTION

It is to be understood that this invention is not limited to the particular methodology, protocols, cell lines, animal species or genera, constructs, and reagents described and as such may vary. It is also to be understood that the terminology used herein is for the purpose of describing particular embodiments only, and is not intended to limit the scope of the present invention which will be limited only by the appended claims.

It must be noted that as used herein and in the appended claims, the singular forms "a," "and," and "the" include plural reference unless the context clearly dictates otherwise. Thus, for example, reference to "a gene" is a reference to one or more genes and includes equivalents thereof known to those skilled in the art, and so forth.

Unless defined otherwise, all technical and scientific terms used herein have the same meaning as commonly understood to one of ordinary skill in the art to which this invention belongs. Although any methods, devices, and materials similar or equivalent to those described herein can be used in the practice or testing of the invention, the preferred methods, devices and materials are now described.

### Definitions

For convenience, the meaning of certain terms and phrases employed in the specification, examples, and appended claims are provided below.

The phrase "a corresponding normal cell of" or "normal cell corresponding to" or "normal counterpart cell of" a diseased cell refers to a normal cell of the same type as that of the diseased cell.

An "address" on an array (e.g., a microarray) refers to a location at which an element, for example, an oligonucleotide, is attached to the solid surface of the array.

The term "agonist," as used herein, is meant to refer to an agent that mimics or up-regulates (e.g., potentiates or supplements) the bioactivity of a protein. An agonist may be a wild-type protein or derivative thereof having at least one bioactivity of the wild-type protein. An agonist may also be a compound that up-regulates expression of a gene or which increases at least one bioactivity of a protein. An agonist can also be a compound which increases the interaction of a polypeptide with another molecule, for example, a target peptide or nucleic acid.

"Amplification," as used herein, relates to the production of additional copies of a nucleic acid sequence. For example, amplification may be carried out using polymerase chain reaction (PCR) technologies which are well known in the art. (see, e.g., Dieffenbach, C. W. and G. S. Dveksler (1995) PCR Primer, A Laboratory Manual, Cold Spring Harbor Press, Plainview, N.Y.)

"Antagonist," as used herein, is meant to refer to an agent that down-regulates (e.g., suppresses or inhibits) at least one bioactivity of a protein. An antagonist may be a compound which inhibits or decreases the interaction between a protein and another molecule, for example, a target peptide or enzyme substrate. An antagonist may also be a compound that down-regulates expression of a gene or which reduces the amount of expressed protein present. For example, a multi-kinase inhibitor is an example of such an antagonist.

The term "antibody," as used herein, is intended to include whole antibodies, for example, of any isotype (IgG, IgA, IgM, IgE, etc.), and includes fragments thereof which are also specifically reactive with a vertebrate (e.g., mammalian) protein. Antibodies may be fragmented using conventional techniques and the fragments screened for utility in the same manner as described above for whole antibodies. Thus, the term includes segments of proteolytically-cleaved or recombinantly-prepared portions of an antibody molecule that are capable of selectively reacting with a certain protein. Non-limiting examples of such proteolytic and/or recombinant fragments include Fab, F(ab')2, Fab', Fv, and single chain antibodies (scFv) containing a V[L] and/or V[H] domain joined by a peptide linker. The scFv's may be covalently or non-covalently linked to form antibodies having two or more binding sites. The subject invention includes polyclonal, monoclonal, or other purified preparations of antibodies and recombinant antibodies.

The terms "array" or "matrix" refer to an arrangement of addressable locations or "addresses" on a device. The locations can be arranged in two-dimensional arrays, three-dimensional arrays, or other matrix formats. The number of locations may range from several to at least hundreds of thousands. Most importantly, each location represents a totally independent reaction site. A "nucleic acid array" refers to an array containing nucleic acid probes, such as oligonucleotides or larger portions of genes. The nucleic acid on the array may be single-stranded. Arrays wherein the probes are oligonucleotides are referred to as "oligonucleotide arrays" or "oligonucleotide chips." A "microarray," also referred to herein as a "biochip" or "biological chip," is an array of regions having a density of discrete regions of at least about 100/cm², or at least about 1000/cm². The regions in a microarray have typical dimensions, for example, diameters, in the range of between about 10-250 µm, and are separated from other regions in the array by about the same distance.

"Biological activity," "bioactivity," "activity," or "biological function," which are used interchangeably, herein mean an effector or antigenic function that is directly or indirectly performed by a polypeptide (whether in its native or denatured conformation), or by any subsequence thereof. Biological activities include binding to polypeptides, binding to other proteins or molecules, activity as a DNA binding protein, as a transcription regulator, ability to bind damaged DNA, etc. A bioactivity can be modulated by directly affecting the subject polypeptide. Alternatively, a bioactivity can be altered by modulating the level of the polypeptide, such as by modulating expression of the corresponding gene.

The term "biological sample," as used herein, refers to a sample obtained from an organism or from components (e.g., cells) of an organism. The sample may be of any biological tissue or fluid. The sample may be a "clinical sample" which is a sample derived from a patient. Such samples include, but are not limited to, sputum, blood, blood cells (e.g., white cells), tissue or fine needle biopsy samples, urine, peritoneal fluid, and pleural fluid, or cells therefrom. Biological samples may also include sections of tissues such as frozen sections taken for histological purposes.

The term "biomarker" or "marker" encompasses a broad range of intra- and extracellular events as well as whole-organism physiological changes. Biomarkers may be represent essentially any aspect of cell function, for example, but not limited to, levels or rate of production of signaling molecules, transcription factors, metabolites, gene transcripts as well as post-translational modifications of proteins. Biomarkers may include whole genome analysis of transcript levels or whole proteome analysis of protein levels and/or modifications.

A biomarker may also refer to a gene or gene product which is up- or down-regulated in a compound-treated, diseased cell of a subject having the disease compared to an untreated diseased cell. That is, the gene or gene product is sufficiently specific to the treated cell that it may be used, optionally with other genes or gene products, to identify, predict, or detect efficacy of a small molecule. Thus, a biomarker is a gene or gene product that is characteristic of efficacy of a compound in a diseased cell or the response of that diseased cell to treatment by the compound.

A nucleotide sequence is "complementary" to another nucleotide sequence if each of the bases of the two sequences match, that is, are capable of forming Watson-Crick base pairs. The term "complementary strand" is used herein interchangeably with the term "complement." The complement of a nucleic acid strand may be the complement of a coding strand or the complement of a non-coding strand.

"Detection agents of genes" refers to agents that can be used to specifically detect the gene or other biological molecules relating to it, for example, RNA transcribed from the gene or polypeptides encoded by the gene. Exemplary detection agents are nucleic acid probes, which hybridize to nucleic acids corresponding to the gene, and antibodies.

"Differential gene expression pattern" between, for example, a normal cell and a disease cell refers to a pattern reflecting the differences in gene expression between a normal cell and a disease cell. A differential gene expression pattern may also be obtained between a cell at one time point and a cell at another time point, or between a cell incubated or contacted with a compound and a cell that has not been incubated with or contacted with the compound.

The term "cancer" includes, but is not limited to, solid tumors, such as cancers of the breast, respiratory tract, brain, reproductive organs, digestive tract, urinary tract, eye, liver, skin, head and neck, thyroid, parathyroid, and their distant metastases. The term also includes lymphomas, sarcomas, and leukemias.

Examples of breast cancer include, but are not limited to, invasive ductal carcinoma, invasive lobular carcinoma, ductal carcinoma *in situ,* and lobular carcinoma *in situ.*

Examples of cancers of the respiratory tract include, but are not limited to, small-cell and non-small-cell lung carcinoma, as well as bronchial adenoma and pleuropulmonary blastoma.

Examples of brain cancers include, but are not limited to, brain stem and hypophtalmic glioma, cerebellar and cerebral astrocytoma, medulloblastoma, ependymoma, as well as neuroectodermal and pineal tumor.

Tumors of the male reproductive organs include, but are not limited to, prostate and testicular cancer. Tumors of the female reproductive organs include, but are not limited to, endometrial, cervical, ovarian, vaginal, and vulvar cancer, as well as sarcoma of the uterus.

Tumors of the digestive tract include, but are not limited to, anal, colon, colorectal, esophageal, gallbladder, gastric, pancreatic, rectal, small-intestine, and salivary gland cancers.

Tumors of the urinary tract include, but are not limited to, bladder, penile, kidney, renal pelvis, ureter, and urethral cancers.

Eye cancers include, but are not limited to, intraocular melanoma and retinoblastoma.

Examples of liver cancers include, but are not limited to, hepatocellular carcinoma (liver cell carcinomas with or without fibrolamellar variant), cholangiocarcinoma (intrahepatic bile duct carcinoma), and mixed hepatocellular cholangiocarcinoma.

Skin cancers include, but are not limited to, squamous cell carcinoma, Kaposi's sarcoma, malignant melanoma, Merkel cell skin cancer, and non-melanoma skin cancer.

Head-and-neck cancers include, but are not limited to, laryngeal / hypopharyngeal / nasopharyngeal / oropharyngeal cancer, and lip and oral cavity cancer.

Lymphomas include, but are not limited to, AIDS-related lymphoma, non-Hodgkin's lymphoma, cutaneous T-cell lymphoma, Hodgkin's disease, and lymphoma of the central nervous system.

Sarcomas include, but are not limited to, sarcoma of the soft tissue, osteosarcoma, malignant fibrous histiocytoma, lymphosarcoma, and rhabdomyosarcoma.

Leukemias include, but are not limited to, acute myeloid leukemia, acute lymphoblastic leukemia, chronic lymphocytic leukemia, chronic myelogenous leukemia, and hairy cell leukemia.

"A diseased cell of cancer" refers to a cell present in subjects having cancer. That is, a cell which is a modified form of a normal cell and is not present in a subject not having cancer, or a cell which is present in significantly higher or lower numbers in subjects having cancer relative to subjects not having cancer.

The term "equivalent" is understood to include nucleotide sequences encoding functionally equivalent polypeptides. Equivalent nucleotide sequences may include sequences that differ by one or more nucleotide substitutions, additions, or deletions, such as allelic variants; and may, therefore, include sequences that differ from the nucleotide sequence of the nucleic acids referred to in Table 1 due to the degeneracy of the genetic code.

The term "expression profile," which is used interchangeably herein with "gene expression profile" and "fingerprint" of a cell refers to a set of values representing mRNA levels of one or more genes in a cell. An expression profile may comprise, for example, values representing expression levels of at least about 2 genes, at least about 5 genes, at least about 10 genes, or at least about 50, 100, 200 or more genes. Expression profiles may also comprise an mRNA level of a gene which is expressed at similar levels in multiple cells and conditions (e.g., a housekeeping gene such as GAPDH).

The term "gene" refers to a nucleic acid sequence that comprises control and coding sequences necessary for the production of a polypeptide or precursor. The polypeptide can be encoded by a full length coding sequence or by any portion of the coding sequence. The gene may be derived in whole or in part from any source known to the art, including a plant, a fungus, an animal, a bacterial genome or episome, eukaryotic, nuclear or plasmid DNA, cDNA, viral DNA, or chemically synthesized DNA. A gene may contain one or more modifications in either the coding or the untranslated regions which could affect the biological activity or the chemical structure of the expression product, the rate of expression, or the manner of expression control. Such modifications include, but are not limited to, mutations, insertions, deletions, and substitutions of one or more nucleotides. The gene may constitute an uninterrupted coding sequence or it may include one or more introns, bound by the appropriate splice junctions.

"hybridization" refers to any process by which a strand of nucleic acid binds with a complementary strand through base pairing. For example, two single-stranded nucleic acids "hybridize" when they form a double-stranded duplex. The region of double-strandedness may include the full-length of one or both of the single-stranded nucleic acids, or all of one single-stranded nucleic acid and a subsequence of the other single-stranded nucleic acid, or the region of double-strandedness may include a subsequence of each nucleic acid. Hybridization also includes the formation of duplexes which contain certain mismatches, provided that the two strands are still forming a double-stranded helix. "Stringent hybridization conditions" refers to hybridization conditions resulting in essentially specific hybridization.

The term "isolated," as used herein, with respect to nucleic acids, such as DNA or RNA, refers to molecules separated from other DNAs or RNAs, respectively, that are present in the natural source of the macromolecule. The term "isolated" as used herein also refers to a nucleic acid or peptide that is substantially free of cellular material, viral material, culture medium when produced by recombinant DNA techniques, or chemical precursors or other chemicals when chemically synthesized. Moreover, an "isolated nucleic acid" may include nucleic acid fragments which are not naturally occurring as fragments and would not be found in the natural state. The term "isolated" is also used herein to refer to polypeptides is meant to encompass both purified and recombinant polypeptides.

As used herein, the terms "label" and "detectable label" refer to a molecule capable of detection, including, but not limited to, radioactive isotopes, fluorophores, chemiluminescent moieties, enzymes, enzyme substrates, enzyme cofactors, enzyme inhibitors, dyes, metal ions, ligands (e.g., biotin or haptens), and the like. The term "fluorescer" refers to a substance or a portion thereof which is capable of exhibiting fluorescence in the detectable range. Particular examples of labels which may be used in the present invention include fluorescein, rhodamine, dansyl, umbelliferone, Texas red, luminol, NADPH, alpha - beta -galactosidase, and horseradish peroxidase.

The phrase "level of expression" refers to the level of mRNA, as well as pre-mRNA nascent transcript(s), transcript processing intermediates, mature mRNA(s), and degradation products, encoded by a gene in the cell. The phrase "level of expression" also refers to the level of protein or polypeptide in a cell.

As used herein, the term "nucleic acid" refers to polynucleotides such as deoxyribonucleic acid (DNA) and, where appropriate, ribonucleic acid (RNA). The term should also be understood to include, as equivalents, analogs of either RNA or DNA made from nucleotide analogs and, as applicable to the embodiment being described, single-stranded (sense or antisense) and double-stranded polynucleotides. Chromosomes, cDNAs, mRNAs, rRNAs, and ESTs are representative examples of molecules that may be referred to as nucleic acids.

The phrase "nucleic acid corresponding to a gene" refers to a nucleic acid that can be used for detecting the gene, for example, a nucleic acid which is capable of hybridizing specifically to the gene.

The phrase "nucleic acid sample derived from RNA" refers to one or more nucleic acid molecules (e.g., RNA or DNA) that may be synthesized from the RNA, and includes DNA produced from methods using PCR (e.g., RT-PCR).

The term "oligonucleotide" as used herein refers to a nucleic acid molecule comprising, for example, from about 10 to about 1000 nucleotides. Oligonucleotides for use in the present invention may be from about 15 to about 150 nucleotides, or from about 150 to about 1000 in length. The oligonucleotide may be a naturally occurring oligonucleotide or a synthetic oligonucleotide. Oligonucleotides may be prepared by the phosphoramidite method (Beaucage and Carruthers, Tetrahedron Lett. 22:1859-62, 1981), or by the triester method (Matteucci, et al., J. Am. Chem. Soc. 103:3185, 1981), or by other chemical methods known in the art.

The term "patient" or "subject" as used herein includes mammals (e.g., humans and animals).

The term "percent identical" refers to sequence identity between two amino acid sequences or between two nucleotide sequences. For example, identity between two sequences may be determined by comparing a particular position in each sequence which may be aligned for purposes of comparison. When an equivalent position in the compared sequences is occupied by the same base or amino acid, then the molecules are identical at that position. When the equivalent site is occupied by the same or a similar amino acid residue (e.g., similar in steric and/or electronic nature), then the molecules may be referred to as homologous (similar) at that position. Expression as a percentage of homology, similarity, or identity refers to a function of the number of identical or similar amino acids at positions shared by the compared sequences. Various alignment algorithms and/or programs may be used including, for example, FASTA, BLAST, or ENTREZ. FASTA and BLAST are available as a part of the GCG sequence analysis package (University of Wisconsin, Madison, Wis.), and may be used with, for example, default settings. ENTREZ is available through the National Center for Biotechnology Information, National Library of Medicine, National Institutes of Health, Bethesda, MD. In one embodiment, the percent identity of two sequences may be determined by the GCG program with a gap weight of 1 (e.g., each amino acid gap is weighted as if it were a single amino acid or nucleotide mismatch between the two sequences). Other techniques for alignment are described in Methods in Enzymology (vol. 266: Computer Methods for Macromolecular Sequence Analysis (1996), ed. Doolittle, Academic Press, Inc., a division of Harcourt Brace & Co., San Diego, California, USA). An alignment program that permits gaps in the sequence may be utilized to align the sequences. For example, the Smith-Waterman is one type of algorithm that permits gaps in sequence alignments (*see*, *e.g*., Meth. Mol. Biol. 70:173-187, 1997). Also, the GAP program using the Needleman and Wunsch alignment method may be utilized to align sequences. An alternative search strategy uses MPSRCH software, which runs on a MASPAR computer. MPSRCH uses a Smith-Waterman algorithm to score sequences on a massively parallel computer. This approach improves the ability to detect distantly related matches, and is especially tolerant of small gaps and nucleotide sequence errors. Nucleic acid-encoded amino acid sequences may be used to search both protein and DNA databases. Databases with individual sequences are described in Methods in Enzymology, ed. Doolittle, *supra.* Databases include, for example, Genbank, EMBL, and DNA Database of Japan (DDBJ).

As used herein, a nucleic acid or other molecule attached to an array is referred to as a "probe" or "capture probe." When an array contains several probes corresponding to one gene, these probes are referred to as a "gene-probe set." A gene-probe set may consist of, for example, about 2 to about 20 probes, from about 2 to about 10 probes, or about 5 probes.

The "profile" of a cell's biological state refers to the levels of various constituents of a cell that are known to change in response to drug treatments and other perturbations of the biological state of the cell. Constituents of a cell include, for example, levels of RNA, levels of protein abundances, or protein activity levels.

The term "protein," "polypeptide," and "peptide" are used interchangeably herein when referring to a gene product.

An expression profile in one cell is "similar" to an expression profile in another cell when the level of expression of the genes in the two profiles are sufficiently similar that the similarity is indicative of a common characteristic, for example, the same type of cell.

"Small molecule," as used herein, refers to a composition with a molecular weight of less than about 5 kD or less than about 4 kD. Small molecules can be nucleic acids, peptides, polypeptides, peptidomimetics, carbohydrates, lipids, or other organic or inorganic molecules. Many pharmaceutical companies have extensive libraries of chemical and/or biological mixtures, often fungal, bacterial, or algal extracts, which can be screened with any of the assays of the invention to identify compounds that modulate a bioactivity.

The term "specific hybridization" of a probe to a target site of a template nucleic acid refers to hybridization of the probe predominantly to the target, such that the hybridization signal can be clearly interpreted. As further described herein, such conditions resulting in specific hybridization vary depending on the length of the region of homology, the GC content of the region, and the melting temperature ("Tm") of the hybrid. Thus, hybridization conditions may vary in salt content, acidity, and temperature of the hybridization solution and the washes.

A "variant" of polypeptide refers to a polypeptide having an amino acid sequence in which one or more amino acid residues is altered. The variant may have "conservative" changes, wherein a substituted amino acid has similar structural or chemical properties (e.g., replacement of leucine with isoleucine). A variant may also have "nonconservative" changes (e.g., replacement of glycine with tryptophan). Analogous minor variations may include amino acid deletions or insertions, or both. Guidance in determining which amino acid residues may be substituted, inserted, or deleted without abolishing biological or immunological activity may be identified using computer programs well known in the art, for example, LASERGENE software (DNASTAR).

The term "variant," when used in the context of a polynucleotide sequence, may encompass a polynucleotide sequence related to that of a particular gene or the coding sequence thereof. This definition may also include, for example, "allelic," "splice," "species," or "polymorphic" variants. A splice variant may have significant identity to a reference molecule, but will generally have a greater or lesser number of polynucleotides due to alternate splicing of exons during mRNA processing. The corresponding polypeptide may possess additional functional domains or an absence of domains. Species variants are polynucleotide sequences that vary from one species to another. The resulting polypeptides generally will have significant amino acid identity relative to each other. A polymorphic variant is a variation in the polynucleotide sequence of a particular gene between individuals of a given species. Polymorphic variants also may encompass "single nucleotide polymorphisms" (SNPs) in which the polynucleotide sequence varies by one base. The presence of SNPs may be indicative of, for example, a certain population, a disease state, or a propensity for a disease state.

### Microarrays for Determining the Level of Expression of Genes

Generally, determining expression profiles with microarrays involves the following steps: (a) obtaining an mRNA sample from a subject and preparing labeled nucleic acids therefrom (the "target nucleic acids" or "targets"); (b) contacting the target nucleic acids with an array under conditions sufficient for the target nucleic acids to bind to the corresponding probes on the array, for example, by hybridization or specific binding; (c) optional removal of unbound targets from the array; (d) detecting the bound targets, and (e) analyzing the results, for example, using computer based analysis methods. As used herein, "nucleic acid probes" or "probes" are nucleic acids attached to the array, whereas "target nucleic acids" are nucleic acids that are hybridized to the array. Each of these steps is described in more detail below.

Nucleic acid specimens may be obtained from an individual to be tested using either "invasive" or "non-invasive" sampling means. A sampling means is said to be "invasive" if it involves the collection of nucleic acids from within the skin or organs of an animal (including murine, human, ovine, equine, bovine, porcine, canine, or feline animal). Examples of invasive methods include blood collection, semen collection, needle biopsy, pleural aspiration, umbilical cord biopsy, etc. Examples of such methods are discussed by Kim, et al., (J. Virol. 66:3879-3882, 1992); Biswas, et al., (Ann. NY Acad. Sci. 590:582-583, 1990); and Biswas, et al., (J. Clin. Microbiol. 29:2228-2233, 1991).

In contrast, a "non-invasive" sampling means is one in which the nucleic acid molecules are recovered from an internal or external surface of the animal. Examples of such "non-invasive" sampling means include, for example, "swabbing," collection of tears, saliva, urine, fecal material, sweat or perspiration, hair, etc.

In one embodiment of the present invention, one or more cells from the subject to be tested are obtained and RNA is isolated from the cells. In one embodiment, a sample of peripheral blood leukocytes (PBLs) cells is obtained from the subject. It is also possible to obtain a cell sample from a subject, and then to enrich the sample for a desired cell type. For example, cells may be isolated from other cells using a variety of techniques, such as isolation with an antibody binding to an epitope on the cell surface of the desired cell type. Where the desired cells are in a solid tissue, particular cells may be dissected, for example, by microdissection or by laser capture microdissection (LCM) (see, e.g., Bonner, et al., Science 278:1481, 1997; Emmert-Buck, et al., Science 274:998, 1996; Fend, et al., Am. J. Path. 154:61, 1999; and Murakami, et al., Kidney Int. 58:1346, 2000).

RNA may be extracted from tissue or cell samples by a variety of methods, for example, guanidium thiocyanate lysis followed by CsCl centrifugation (Chirgwin, et al., Biochemistry 18:5294-5299, 1979). RNA from single cells may be obtained as described in methods for preparing cDNA libraries from single cells (*see, e.g*., Dulac, Curr. Top. Dev. Biol. 36:245, 1998; Jena, et al., J. Immunol. Methods 190:199, 1996).

The RNA sample can be further enriched for a particular species. In one embodiment, for example, poly(A)+ RNA may be isolated from an RNA sample. In particular, poly-T oligonucleotides may be immobilized on a solid support to serve as affinity ligands for mRNA. Kits for this purpose are commercially available, for example, the MessageMaker kit (Life Technologies, Grand Island, NY).

In one embodiment, the RNA population may be enriched for sequences of interest, such as the genes described in Table 1 (e.g., SEQ ID NOs: 1-18). Enrichment may be accomplished, for example, by primer-specific cDNA synthesis, or multiple rounds of linear amplification based on cDNA synthesis and template-directed *in vitro* transcription (*see, e.g*., Wang, et al., Proc. Natl. Acad. Sci. USA 86:9717, 1989; Dulac, et al., *supra;* Jena, et al., *supra*).

The population of RNA, enriched or not in particular species or sequences, may be further amplified. Such amplification is particularly important when using RNA from a single cell or a few cells. A variety of amplification methods are suitable for use in the methods of the present invention, including, for example, PCR; ligase chain reaction (LCR) (*see, e.g.*, Wu and Wallace, Genomics 4:560, 1989; Landegren, et al., Science 241:1077, 1988); self-sustained sequence replication (SSR) (*see, e.g*., Guatelli, et al., Proc. Natl. Acad. Sci. USA 87:1874, 1990); nucleic acid based sequence amplification (NASBA) and transcription amplification (*see, e.g.,* Kwoh, et al., Proc. Natl. Acad. Sci. USA 86:1173,1989). Methods for PCR technology are well known in the art (*see*, *e.g.,* PCR Technology: Principles and Applications for DNA Amplification (ed. H. A. Erlich, Freeman Press, N.Y., N.Y., 1992); PCR Protocols: A Guide to Methods and Applications (eds. Innis, et al., Academic Press, San Diego, Calif., 1990); Mattila, et al., Nucleic Acids Res. 19:4967, 1991; Eckert, et al., PCR Methods and Applications 1:17, 1991; PCR (eds. McPherson, et al., IRL Press, Oxford); and U.S. Pat. No. 4,683,202). Methods of amplification are described, for example, by Ohyama, et al., (BioTechniques 29:530, 2000); Luo, et al., (Nat. Med. 5:117, 1999); Hegde, et al., (BioTechniques 29:548, 2000); Kacharmina, et al., (Meth. Enzymol. 303:3, 1999); Livesey, et al., Curr. Biol. 10:301, 2000); Spirin, et al., (invest. Ophtalmol. Vis. Sci. 40:3108, 1999); and Sakai, et al., (Anal. Biochem. 287:32, 2000). RNA amplification and cDNA synthesis may also be conducted in cells *in situ* (*see, e.g*., Eberwine, et al. Proc. Natl. Acad. Sci. USA 89:3010, 1992).

Generally, the target molecules will be labeled to permit detection of hybridization of the target molecules to a microarray. That is, the probe may comprise a member of a signal producing system and thus, is detectable, either directly or through combined action with one or more additional members of a signal producing system. Examples of directly detectable labels include isotopic and fluorescent moieties incorporated, usually by a covalent bond, into a moiety of the probe, such as a nucleotide monomeric unit (e.g., dNMP of the primer), or a photoactive or chemically active derivative of a detectable label which can be bound to a functional moiety of the probe molecule.

Nucleic acids may be labeled during or after enrichment and/or amplification of RNAs. For example, reverse transcription may be carried out in the presence of a dNTP conjugated to a detectable label, for example, a fluorescently labeled dNTP. In another embodiment, the cDNA or RNA probe may be synthesized in the absence of detectable label and may be labeled subsequently, for example, by incorporating biotinylated dNTPs or rNTP, or some similar means (e.g., photo-cross-linking a psoralen derivative of biotin to RNAs), followed by addition of labeled streptavidin (e.g., phycoerythrin-conjugated streptavidin) or the equivalent.

Fluorescent moieties or labels of interest include coumarin and its derivatives (e.g., 7-amino-4-methylcoumarin, aminocoumarin); bodipy dyes such as Bodipy FL and cascade blue; fluorescein and its derivatives (e.g., fluorescein isothiocyanate, Oregon green); rhodamine dyes (e.g., Texas red, tetramethylrhodamine); eosins and erythrosins; cyanine dyes (e.g., Cy2, Cy3, Cy3.5, Cy5, Cy5.5, Cy7); FluorX, macrocyclic chelates of lanthanide ions (e.g., quantum dye™); fluorescent energy transfer dyes such as thiazole orange-ethidium heterodimer, TOTAB, dansyl, etc. Individual fluorescent compounds which have functionalities for linking to an element desirably detected in an apparatus or assay of the invention, or which may be modified to incorporate such functionalities may also be utilized (*see, e.g.,* Kricka, 1992, Nonisotopic DNA Probe Techniques, Academic Press San Diego, Calif.).

Chemiluminescent labels include luciferin and 2,3-dihydrophthalazinediones, for example, luminol.

Labels may also be members of a signal producing system that act in concert with one or more additional members of the same system to provide a detectable signal. Illustrative of such labels are members of a specific binding pair, such as ligands, for example, biotin, fluorescein, digoxigenin, antigen, polyvalent cations, chelator groups and the like. Members may specifically bind to additional members of the signal producing system, and the additional members may provide a detectable signal either directly or indirectly, for example, an antibody conjugated to a fluorescent moiety or an enzymatic moiety capable of converting a substrate to a chromogenic product (e.g., alkaline phosphatase conjugate antibody and the like).

Additional labels of interest include those that provide a signal only when the probe with which it is associated is specifically bound to a target molecule. Such labels include "molecular beacons" as described in Tyagi and Kramer (Nature Biotech. 14:303, 1996) and EP 0 070 685 B1. Other labels of interest include those described in U.S. Patent No. 5,563,037; WO 97/17471; and WO 97/17076.

In other embodiments, the target nucleic acid may not be labeled. In this case, hybridization may be determined, for example, by plasmon resonance (*see, e.g*., Thiel, et al. Anal. Chem. 69:4948, 1997).

In one embodiment, a plurality (e.g., 2, 3, 4, 5, or more) of sets of target nucleic acids are labeled and used in one hybridization reaction ("multiplex" analysis). For example, one set of nucleic acids may correspond to RNA from one cell and another set of nucleic acids may correspond to RNA from another cell. The plurality of sets of nucleic acids may be labeled with different labels, for example, different fluorescent labels (e.g., fluorescein and rhodamine) which have distinct emission spectra so that they can be distinguished. The sets may then be mixed and hybridized simultaneously to one microarray (*see, e.g*., Shena, et al., Science 270:467-470, 1995).

Examples of distinguishable labels for use when hybridizing a plurality of target nucleic acids to one array are well known in the art and include: two or more different emission wavelength fluorescent dyes such as Cy3 and Cy5; combination of fluorescent proteins and dyes such as phicoerythrin and Cy5; two or more isotopes with different energy of emission such as ³²P and ³³P; gold or silver particles with different scattering spectra; labels which generate signals under different treatment conditions such as temperature, pH, treatment with additional chemical agents, etc.; or generate signals at different time points after treatment. Using one or more enzymes for signal generation allows for the use of an even greater variety of distinguishable labels, based on different substrate specificity of enzymes (e.g., alkaline phosphatase/peroxidase).

The quality of labeled nucleic acids may be evaluated prior to hybridization to an array. In one embodiment, the GeneChip^{®} Test3 Array from Affymetrix (Santa Clara, CA) may be used for that purpose. This array contains probes representing a subset of characterized genes from several organisms including mammals. Thus, the quality of a labeled nucleic acid sample can be determined by hybridization of a fraction of the sample to an array.

Microarrays for use according to the invention include one or more probes of genes characteristic of small molecule efficacy. In one embodiment, the microarray comprises probes corresponding to one or more of genes selected from the group consisting of genes which are up-regulated in cancer and genes which are down-regulated in cancer. The microarray may comprise, for example, probes corresponding to at least 2, at least 5, at least 10, at least 100 or more characteristic of small molecule efficacy. The microarray may comprise probes corresponding to each gene or gene product listed in Table 1.

There may be one or more than one probe corresponding to each gene on a microarray. For example, a microarray may contain from 2 to 20 probes corresponding to one gene or about 5 to 10. The probes may correspond to the full-length RNA sequence or complement thereof of genes characteristic of small molecule efficacy, or the probe may correspond to a portion thereof, which portion is of sufficient length to permit specific hybridization. Such probes may comprise from about 50 nucleotides to about 100, 200, 500, or 1000 nucleotides or more than 1000 nucleotides. As further described herein, microarrays may contain oligonucleotide probes, consisting of about 10 to 50 nucleotides, about 15 to 30 nucleotides, or about 20-25 nucleotides. The probes are may be single-stranded and will have sufficient complementarity to its target to provide for the desired level of sequence specific hybridization.

Typically, the arrays used in the present invention will have a site density of greater than 100 different probes per cm². The arrays may have a site density of, for example, greater than 500/cm², greater than about 1000/cm², or greater than about 10,000/cm². The arrays may have, for example, more than 100 different probes on a single substrate, greater than about 1000 different probes, greater than about 10,000 different probes, or greater than 100,000 different probes on a single substrate.

A number of different microarray configurations and methods for their production are known to those of skill in the art and are disclosed in U.S. Patent Nos: 5,242,974; 5,384,261; 5,405,783; 5,412,087; 5,424,186; 5,429,807; 5,436,327; 5,445,934; 5,556,752; 5,405,783; 5,412,087; 5,424,186; 5,429,807; 5,436,327; 5,472,672; 5,527,681; 5,529,756; 5,545,531; 5,554,501; 5,561,071; 5,571,639; 5,593,839; 5,624,711; 5,700,637; 5,744,305; 5,770,456; 5,770,722; 5,837,832; 5,856,101; 5,874,219; 5,885,837; 5,919,523; 6,022,963; 6,077,674; and 6,156,501; Shena, et al., Tibtech 16:301, 1998; Duggan, et al., Nat. Genet. 21:10, 1999; Bowtell, et al., Nat. Genet. 21:25, 1999; Lipshutz, et al., 21 Nature Genet. 20-24, 1999; Blanchard, et al., 11 Biosensors and Bioelectronics, 687-90, 1996; Maskos, et al., 21 Nucleic Acids Res. 4663-69, 1993; Hughes, et al., Nat. Biotechol. 19:342, 2001. Patents describing methods of using arrays in various applications include: U.S. Pat. Nos. 5,143,854; 5,288,644; 5,324,633; 5,432,049; 5,470,710; 5,492,806; 5,503,980; 5,510,270; 5,525,464; 5,547,839; 5,580,732; 5,661,028; 5,848,659; and 5,874,219.

Arrays may also include control and reference nucleic acids. Control nucleic acids include, for example, prokaryotic genes such as bioB, bioC and bioD, cre from P1 bacteriophage or polyA controls, such as dap, lys, phe, thr, and trp. Reference nucleic acids allow the normalization of results from one experiment to another and the comparison of multiple experiments on a quantitative level. Exemplary reference nucleic acids include housekeeping genes of known expression levels, for example, GAPDH, hexokinase, and actin.

In one embodiment, an array of oligonucleotides may be synthesized on a solid support. Exemplary solid supports include glass, plastics, polymers, metals, metalloids, ceramics, organics, etc. Using chip masking technologies and photoprotective chemistry, it is possible to generate ordered arrays of nucleic acid probes. These arrays, which are known, for example, as "DNA chips" or very large scale immobilized polymer arrays ("VLSIPS^{™}" arrays), may include millions of defined probe regions on a substrate having an area of about 1 cm² to several cm², thereby incorporating from a few to millions of probes (see, e.g., U.S. Patent No. 5,631,734).

A nucleic acid probe may be at least, for example, about 10, 15, 20, 25, 30, 50, 100 or more nucleotides, and may comprise the full-length gene. For example, probes may be those that hybridize specifically to the genes listed in Table 1.

Nucleic acid probes may be obtained, for example, by PCR amplificartion of gene segments from genomic, cDNA (e.g., RT-PCR), or cloned sequences. cDNA probes may be prepared according to methods known in the art and further described herein, for example, by reverse-transcription PCR (RT-PCR) of RNA using sequence specific primers. Sequences of genes or cDNA from which probes are generated may be obtained, for example, from GenBank, other public databases, or publications.

Oligonucleotide probes may also be synthesized by standard methods known in the art, for example, by automated DNA synthesizer or any other chemical method. As an example, phosphorothioate oligonucleotides may be synthesized by the method of Stein, et al., (Nucl. Acids Res. 16:3209, 1988), and methylphosphonate oligonucleotides may be prepared by controlled pore glass polymer supports (see, e.g., Sarin, et al., Proc. Natl. Acad. Sci. U.S.A. 85:7448-7451, 1988). In another embodiment, the oligonucleotide may be a 2'-)-methylribonucleotide (Inoue, et al., Nucl. Acids Res. 15:6131-6148, 1987), or a chimeric RNA-DNA analog (Inoue, et al., FEBS Lett. 215:327-330, 1987).

Nucleic acid probes may be natural nucleic acids or chemically modified nucleic acids (e.g., composed of nucleotide analogs); however, the probes should possess activated hydroxyl groups compatible with the linking chemistry. The protective groups may be photolabile, or the protective groups may be labile under certain chemical conditions (e.g., acid). The surface of the solid support may contain a composition that generates acids upon exposure to light. Thus, exposure of a region of the substrate to light generates acids in that region that remove the protective groups in the exposed region. Also, the synthesis method may use 3'- protected 5'-O-phosphoramidite-activated deoxynucleoside. In this case, the oligonucleotide is synthesized in the 5' to 3' direction, which results in a free 5' end.

In one embodiment of the present invention, oligonucleotides of an array may be synthesized using a 96-well automated multiplex oligonucleotide synthesizer (A.M.O.S.) that is capable of producing thousands of oligonucleotides (see, e.g., Lashkari, et al., Proc. Natl. Acad. Sci. USA 93: 7912, 1995).

To compare expression levels, labeled nucleic acids may be contacted with the array under conditions sufficient for binding between the target nucleic acid and the probe on the array. In one embodiment, the hybridization conditions may be selected to provide for the desired level of hybridization specificity; that is, conditions sufficient for hybridization to occur between the labeled nucleic acids and probes on the microarray.

Hybridization may be carried out in conditions permitting essentially specific hybridization. The length and GC content of the nucleic acid will determine the thermal melting point and thus, the hybridization conditions necessary for obtaining specific hybridization of the probe to the target nucleic acid. These factors are well known to a person of skill in the art, and may also be tested in assays. An extensive guide to nucleic acid hybridization may be found in Tijssen, et al. (Laboratory Techniques in Biochemistry and Molecular Biology, Vol. 24: Hybridization With Nucleic Acid Probes, P. Tijssen, ed. Elsevier, N.Y., (1993)). Generally, stringent conditions may be selected to be about 5°C lower than the thermal melting point (Tm) for the specific sequence at a defined ionic strength and pH. The Tm is the temperature (under defined ionic strength and pH) at which 50% of the target sequence hybridizes to a perfectly matched probe. Highly stringent conditions may be selected to be equal to the Tm point for a particular probe. Sometimes the term "dissociation temperature" (Td) is used to define the temperature at which at least half of the probe dissociates from a perfectly matched target nucleic acid. In any case, a variety of techniques for estimating the Tm or Td are available, and generally are described in Tijssen, *supra.* Typically, G-C base pairs in a duplex are estimated to contribute about 3°C to the Tm, while A-T base pairs are estimated to contribute about 2°C, up to a theoretical maximum of about 80-100°C. However, more sophisticated models of Tm and Td are available in which G-C stacking interactions, solvent effects, the desired assay temperature, and the like are taken into account.

In one embodiment, non-specific binding or background signal may be reduced by the use of a detergent (e.g, C-TAB) or a blocking reagent (e.g., sperm DNA, cot-1 DNA, etc.) during the hybridization. In one embodiment, the hybridization may be performed in the presence of about 0.5 mg/ml DNA (e.g., herring sperm DNA). The use of blocking agents in hybridization is well known to those of skill in the art (*see, e.g*., Tijssen, *supra*).

If the target sequences are detected using the same label, different arrays may be employed for each physiological source or the same array may be screened multiple times. The above methods may be varied to provide for multiplex analysis by employing different and distinguishable labels for the different target populations (e.g., different physiological sources). According to this multiplex method, the same array may be used at the same time for each of the different target populations.

The methods described above result in the production of hybridization patterns of labeled target nucleic acids on the array surface. The resultant hybridization patterns of labeled nucleic acids may be visualized or detected in a variety of ways, with the particular manner of detection selected based on the particular label of the target nucleic acid. Representative detection means include scintillation counting, autoradiography, fluorescence measurement, colorimetric measurement, light emission measurement, light scattering, and the like.

One such method of detection utilizes an array scanner that is commercially available (Affymetrix, Santa Clara, CA), for example, the 417^{™} Arrayer, the 418^{™} Array Scanner, or the Agilent GeneArray^{™}Scanner. This scanner is controlled from a system computer with an interface and easy-to-use software tools. The output may be directly imported into or directly read by a variety of software applications. Scanning devices are described in, for example, U.S. Patent Nos. 5,143,854 and 5,424,186.

For fluorescent labeled probes, the fluorescence emissions at each site of a transcript array may be detected by scanning confocal laser microscopy. Alternatively, a laser may be used that allows simultaneous specimen illumination at wavelengths specific to the two fluorophores and emissions from the two fluorophores may be analyzed simultaneously (*see, e.g.,* Shalon, et al., Genome Res. 6:639-645, 1996). For example, the arrays may be scanned with a laser fluorescent scanner with a computer controlled X-Y stage and a microscope objective. Fluorescence laser scanning devices are described in Shalon, et al., *supra.*

Following the data gathering operation, the data will typically be reported to a data analysis operation. To facilitate the sample analysis operation, the data obtained by the reader from the device may be analyzed using a digital computer. Typically, the computer will be appropriately programmed for receipt and storage of the data from the device, as well as for analysis and reporting of the data gathered, for example, subtraction of the background, deconvolution of multi-color images, flagging or removing artifacts, verifying that controls have performed properly, normalizing the signals, interpreting fluorescence data to determine the amount of hybridized target, normalization of background and single base mismatch hybridizations, and the like.

In one embodiment, a system comprises a search function that allows one to search for specific patterns, for example, patterns relating to differential gene expression, for example, between the expression profile of a cancer cell and the expression profile of a counterpart normal cell in a subject. For example, a system allows one to search for patterns of gene expression between more than two samples.

Various algorithms are available for analyzing gene expression profile data, for example, the type of comparisons to perform. In certain embodiments, it is desirable to group genes that are co-regulated. This allows for the comparison of large numbers of profiles. One embodiment for identifying such groups of genes involves clustering algorithms (for reviews of clustering algorithms, *see, e.g*., Fukunaga, 1990, Statistical Pattern Recognition, 2nd Ed., Academic Press, San Diego; Everitt, 1974, Cluster Analysis, London: Heinemann Educ. Books; Hartigan, 1975, Clustering Algorithms, New York: Wiley; Sneath and Sokal, 1973, Numerical Taxonomy, Freeman; Anderberg, 1973, Cluster Analysis for Applications, Academic Press: New York).

Clustering may be based on other characteristics of the genes, for example, their level of expression (*see, e.g*., U.S. Patent No. 6,203,987), or permit clustering of time curves (*see, e.g.* U.S. Patent No. 6,263,287). Examples of clustering algorithms include K-means clustering and hierarchical clustering. Clustering may also be achieved by visual inspection of gene expression data using a graphical representation of the data (e.g. a "heat map"). An example of software which contains clustering algorithms and a means to graphically represent gene expression data is Spotfire DecisionSite (Spotfire, Inc., Somerville, Massachusetts and Göteborg, Sweden).

Comparison of the expression levels of one or more genes characteristic of small molecule efficacy with reference expression levels, for example, expression levels in diseased cells of cancer or in normal counterpart cells, may be conducted using computer systems. In one embodiment, expression levels may be obtained from two cells and these two sets of expression levels may be introduced into a computer system for comparison. For example, one set of expression levels is entered into a computer system for comparison with values that are already present in the computer system, or in computer-readable form that is then entered into the computer system.

In one embodiment, the computer system may also contain a database comprising values representing levels of expression of one or more genes characteristic of small molecule efficacy. The database may contain one or more expression profiles of genes characteristic of small molecule efficacy in different cells.

In another embodiment, the invention provides a computer-readable form of the gene expression profile data, or of values corresponding to the level of expression of at least one gene characteristic of cancer in a diseased cell. The values may be mRNA expression levels obtained from experiments, for example, microarray analysis. The values may also be mRNA levels normalized relative to a reference gene whose expression is constant in numerous cells under numerous conditions (e.g., GAPDH). In other embodiments, the values in the computer may be ratios of, or differences between, normalized or non-normalized mRNA levels in different samples.

In one embodiment, the expression profiles expression profiles from cancer cells of one or more subjects, which cells are treated *in vivo* or *in vitro* with a drug, for example, an multi-kinase inhibitor. Expression data of a cell of a subject treated *in vitro* or *in vivo* with the drug is entered into a computer and the computer is instructed to compare the data entered to the data in the computer, and to provide results indicating whether the expression data input into the computer are more similar to those of a cell of a subject that is responsive to the drug or more similar to those of a cell of a subject that is not responsive to the drug. Thus, the results indicate whether the subject is likely to respond to the treatment with the drug or unlikely to respond to it.

The invention also provides a machine-readable or computer-readable medium including program instructions for performing the following steps: (i) comparing a plurality of values corresponding to expression levels of one or more genes characteristic of small molecule efficacy in a query cell with a database including records comprising reference expression or expression profile data of one or more reference cells and an annotation of the type of cell; and (ii) indicating to which cell the query cell is most similar based on similarities of expression profiles. The reference cells may be cells from subjects at different stages of cancer. The reference cells may also be cells from subjects responding or not responding to a particular drug treatment and optionally incubated *in vitro* or *in vivo* with the drug.

The reference cells may also be cells from subjects responding or not responding to several different treatments, and the computer system indicates a preferred treatment for the subject. Accordingly, the invention provides a method for selecting a therapy for a patient having cancer, the method comprising: (i) providing the level of expression of one or more genes characteristic of small molecule efficacy in a diseased cell of the patient; (ii) providing a plurality of reference profiles, each associated with a therapy, wherein the subject expression profile and each reference profile has a plurality of values, each value representing the level of expression of a gene characteristic of cancer; and (iii) selecting the reference profile most similar to the subject expression profile, to thereby select a therapy for said patient. In one embodiment, step (iii) may be performed by a computer. The most similar reference profile may be selected by weighing a comparison value of the plurality using a weight value associated with the corresponding expression data.

The relative abundance of an mRNA in two biological samples may be scored as a perturbation and its magnitude determined (i.e., the abundance is different in the two sources of mRNA tested), or as not perturbed (i.e., the relative abundance is the same). In various embodiments, a difference between the two sources of RNA is scored as a perturbation. Perturbations may be used by a computer for calculating and expression comparisons.

### Drug Design Using Microarrays

The invention also provides methods for designing and optimizing drugs for cancer, for example, those which have been identified as described herein. In one embodiment, compounds may be screened by comparing the expression level of one or more genes characteristic of small molecule efficacy following incubation of a diseased cell of cancer or similar cell with the test compound. In another embodiment, the expression level of the genes may be determined using microarrays, and comparing the gene expression profile of a cell in response to the test compound with the gene expression profile of a normal cell corresponding to a diseased cell of cancer (a "reference profile"). In a further embodiment, the expression profile may also be compared to that of a diseased cell of cancer. The comparisons may be done by introducing the gene expression profile data of the cell treated with drug into a computer system comprising reference gene expression profiles, which are stored in a computer readable form, using appropriate algorithms. Test compounds may be screened for those that alter the level of expression of genes characteristic of small molecule efficacy. Such compounds, that is, compounds which are capable of normalizing the expression of essentially all genes characteristic of small molecule efficacy, are candidate therapeutics.

The efficacy of the compounds may then be tested in additional *in vitro* and *in vivo* assays, and in animal models (e.g., xenograft model). The test compound may be administered to the test animal, and one or more symptoms of the disease may be monitored for improvement of the condition of the animal. Expression of one or more genes characteristic of small molecule efficacy may also be measured before and after administration of the test compound to the animal. A normalization of the expression of one or more of these genes is indicative of the efficiency of the compound for treating cancer in the animal.

In the clinical setting, obtaining human-derived samples of tissue exhibiting cancer may be difficult, if not prohibitive. Therefore, identification of gene expression changes indicative of efficacy of a therapeutic compound may be determined in a more easily accessible, surrogate cell population, for example, peripheral blood leukocytes (PBLs). This method may be performed either in a human or animal model system. In one embodiment, a test compound may be administered to the test animal (either normal or cancer-containing) at the same doses that have been observed to be efficacious in treating cancer in that animal model. Blood may be drawn from the animal at various time points (e.g., 1, 4, 7, and 24 hours following the first, mid-point, and last day of a regimen of multiple day dosing). Animals dosed with vehicle may be used as controls. RNA may be isolated from PBLs, and can be used to generate probes for hybridization to microarrays. The hybridization results may then be analyzed using computer programs and databases, as described above. The resulting expression profile may be compared directly to the analogous profile from the treated cancer tissue for similarities or simply correlated with efficacy (e.g., in terms of doses and time points) in the animal model.

In another embodiment, human blood may be treated *ex vivo* with a therapeutic compound at a dose consistent with the therapeutic dose in the animal model, or at a dose that is consistent with known plasma levels of the therapeutic dose in the animal model. The blood may be treated (e.g., rocking at 37°C) with the therapeutic compound immediately, or after some period of incubation time (e.g., 24 hours) to allow for gene expression to reequilibrate after the blood draw. The blood may also be treated with the therapeutic compound for various timepoints (e.g., 4 and 24 hours), and then PBL RNA isolated and used to create a probe for hybridization to a microarray. A compound solubilization agent (e.g., DMSO) may be used as a control. The resulting expression profile may be compared directly to the analogous profile from the treated cancer tissue for similarities or simply correlated with efficacy (e.g., in terms of doses and time points) in the animal model.

The toxicity of the candidate therapeutic compound may be evaluated, for example, by determining whether the compound induces the expression of genes known to be associated with a toxic response. Expression of such toxicity related genes may be determined in different cell types, for example, those that are known to express the genes. In fact, alterations in gene expression may serve as a more sensitive marker of human toxicity than routine preclinical safety studies. Microarrays may be used for detecting changes in the expression of genes known to be associated with a toxic response. It may be possible to perform proof of concept studies demonstrating that changes in gene expression levels may predict toxic events that were not identified by routine preclinical safety testing (*see, e.g*., Huang, et al., Toxicol. Sci. 63:196-207, 2001; Waring, et al., Toxicol. Appl. Pharamacol. 175:28-42, 2001).

Drug screening may be performed by adding a test compound to a sample of cells, and monitoring the effect. A parallel sample which does not receive the test compound may also be monitored as a control. The treated and untreated cells are then compared by any suitable phenotypic criteria, including but not limited to microscopic analysis, viability testing, ability to replicate, histological examination, the level of a particular RNA or polypeptide associated with the cells, the level of enzymatic activity expressed by the cells or cell lysates, and the ability of the cells to interact with other cells or compounds. Differences between treated and untreated cells indicates effects attributable to the test compound.

Desirable effects of a test compound include an effect on any phenotype that was conferred by the cancer-associated marker nucleic acid sequence. Examples include a test compound that limits the overabundance of mRNA, limits production of the encoded protein, or limits the functional effect of the protein. The effect of the test compound would be apparent when comparing results between treated and untreated cells.

### Diagnostic and Prognostic Assays

The present invention provides nucleic acid sequences which are differentially regulated in cancer, and a method for identifying such sequences. The present invention provides a method for identifying a nucleotide sequence which is differentially regulated in a subject with cancer, comprising: hybridizing a nucleic acid sample corresponding to RNA obtained from the subject to a nucleic acid sample comprising one or more nucleic acid molecules of known identity; and measuring the hybridization of the nucleic acid sample to the one or more nucleic acid molecules of known identity, wherein a difference in the hybridization of the nucleic acid sample to the one or more nucleic acid molecules of known identity relative to a nucleic acid sample obtained from a subject without cancer is indicative of the differential expression of the nucleotide sequence in a subject with cancer.

Generally, the present invention provides a method for identifying nucleic acid sequences which are differentially regulated in a subject with cancer comprising isolating messenger RNA from a subject, generating cRNA from the mRNA sample, hybridizing the cRNA to a microarray comprising a plurality of nucleic acid molecules stably associated with discrete locations on the array, and identifying patterns of hybridization of the cRNA to the array. According to the present invention, a nucleic acid molecule which hybridizes to a given location on the array is said to be differentially regulated if the hybridization signal is, for example, higher or lower than the hybridization signal at the same location on an identical array hybridized with a nucleic acid sample obtained from a subject that does not have cancer.

Expression patterns may be used to derive a panel of biomarkers that can be used to predict the efficacy of drug treatment in the patients. The biomarkers may consist of gene expression levels from microarray experiments on RNA isolated from biological samples, RNA isolated from frozen samples of tumor biopsies, or mass spectrometry-derived protein masses in the serum.

Although the precise mechanism for data analysis will depend upon the exact nature of the data, a typical procedure for developing a panel of biomarkers is as follows. The data (gene expression levels or mass spectra) are collected for each patient prior to treatment. As the study progresses, the patients are classified according to their response to the drug treatment; either as efficacious or non-efficacious. Multiple levels of efficacy can be accommodated in a data model, but a binary comparison is considered optimal, particularly if the patient population is less than several hundred. Assuming adequate numbers of patients in each class, the protein and/or gene expression data may be analyzed by a number of techniques known in the art. Many of the techniques are derived from traditional statistics as well from the field of machine learning. These techniques serve two purposes:
1. Reduce the dimensionality of data - In the case of mass spectra or gene expression microarrays, data is reduced from many thousands of individual data points to, for example, about three to ten. The reduction is based upon the predictive power of the data points when taken as a set.
2. Training - These three to ten data points are then used to train multiple machine learning algorithms which then "learn" to recognize, in this case, patterns of protein masses or gene expression which distinguish efficacious drug treatment from non-efficacious. All patient samples can be used to train the algorithms.

The resulting trained algorithms are then tested in order to measure their predictive power. Typically, when less than many hundreds of training examples are available, some form of cross-validation is performed. To illustrate, consider a ten-fold cross validation. In this case, patient samples are randomly assigned to one of ten bins. In the first round of validation the samples in nine of the bins are used for training and the remaining samples in the tenth bin are used to test the algorithm. This is repeated an additional nine times, each time leaving out the samples in a different bin for testing. The results (correct predictions and errors) from all ten rounds are combined and the predictive power is then assessed. Different algorithms, as well as different panels, may be compared in this way for this study. The "best" algorithm/panel combination will then be selected. This "smart" algorithm may then be used in future studies to select the patients that are most likely to respond to treatment.

Many algorithms benefit from additional information taken for the patients. For example, gender or age could be used to improve predictive power. Also, data transformations such as normalization and smoothing may be used to reduce noise. Because of this, a large number of algorithms may be trained using many different parameters in order to optimize the outcome. If predictive patterns exist in the data, it is likely that an optimal, or near-optimal, "smart" algorithm can be developed. If more patient samples become available, the algorithm can be retrained to take advantage of the new data.

As an example using mass spectrometry, plasma may be applied to a hydrophobic SELDI-target, washed extensively in water, and analyzed by the SELDI-Tof mass spectrometer. This may be repeated on 100 or more patient samples. The protein profiles resulting from the intensities of some 16,000 m/z values in each sample would be statistically analyzed in order to identify sets of specific m/z values that are predictive of drug efficacy. Identical experiments using other SELDI-targets, such as ion-exchange or IMAC surfaces, could also be conducted. These will capture different subsets of the proteins present in plasma. Furthermore, the plasma may be denatured and prefractionated prior to application onto the SELDI target.

The present invention provides methods for determining whether a subject is at risk for developing a disease or condition characterized by unwanted cell proliferation by detecting biomarkers, that is, nucleic acids and/or polypeptide markers for cancer.

In clinical applications, human tissue samples may be screened for the presence and/or absence of biomarkers identified herein. Such samples could consist of needle biopsy cores, surgical resection samples, lymph node tissue, or serum. For example, these methods include obtaining a biopsy, which is optionally fractionated by cryostat sectioning to enrich tumor cells to about 80% of the total cell population. In certain embodiments, nucleic acids extracted from these samples may be amplified using techniques well known in the art. The levels of selected markers detected would be compared with statistically valid groups of metastatic, non-metastatic malignant, benign, or normal tissue samples.

In one embodiment, the diagnostic method comprises determining whether a subject has an abnormal mRNA and/or protein level of the biomarkers, such as by Northern blot analysis, reverse transcription-polymerase chain reaction (RT-PCR), *in situ* hybridization, immunoprecipitation, Western blot hybridization, or immunohistochemistry. According to the method, cells may be obtained from a subject and the levels of the biomarkers, protein, or mRNA level, are determined and compared to the level of these markers in a healthy subject. An abnormal level of the biomarker polypeptide or mRNA levels is likely to be indicative of cancer.

In one embodiment, the method comprises using a nucleic acid probe to determine the presence of cancerous cells in a tissue from a patient. Specifically, the method comprises:
1. providing a nucleic acid probe comprising a nucleotide sequence, for example, at least 10, 15, 25 or 40 nucleotides, and up to all or nearly all of the coding sequence which is complementary to a portion of the coding sequence of a nucleic acid sequence and is differentially expressed in tumors cells;
2. obtaining a tissue sample from a patient potentially comprising cancerous cells;
3. providing a second tissue sample containing cells substantially all of which are non-cancerous;
4. contacting the nucleic acid probe under stringent conditions with RNA of each of said first and second tissue samples (e.g., in a Northern blot or *in situ* hybridization assay); and
5. comparing (a) the amount of hybridization of the probe with RNA of the first tissue sample, with (b) the amount of hybridization of the probe with RNA of the second tissue sample; wherein a statistically significant difference in the amount of hybridization with the RNA of the first tissue sample as compared to the amount of hybridization with the RNA of the second tissue sample is indicative of the presence of cancerous cells in the first tissue sample.

In one aspect, the method comprises *in situ* hybridization with a probe derived from a given marker nucleic acid sequence. The method comprises contacting the labeled hybridization probe with a sample of a given type of tissue potentially containing cancerous or pre-cancerous cells as well as normal cells, and determining whether the probe labels some cells of the given tissue type to a degree significantly different than the degree to which it labels other cells of the same tissue type.

Also within the invention is a method of determining the phenotype of a test cell from a given human tissue, for example, whether the cell is (a) normal, or (b) cancerous or precancerous, by contacting the mRNA of a test cell with a nucleic acid probe, for example, at least about 10, 15, 25, or 40 nucleotides, and up to all or nearly all of a sequence which is complementary to a portion of the coding sequence of a nucleic acid sequence, and which is differentially expressed in tumor cells as compared to normal cells of the given tissue type; and determining the approximate amount of hybridization of the probe to the mRNA, an amount of hybridization either more or less than that seen with the mRNA of a normal cell of that tissue type being indicative that the test cell is cancerous or pre-cancerous.

Alternatively, the above diagnostic assays may be carried out using antibodies to detect the protein product encoded by the marker nucleic acid sequence. Accordingly, in one embodiment, the assay would include contacting the proteins of the test cell with an antibody specific for the gene product of a nucleic acid, the marker nucleic acid being one which is expressed at a given control level in normal cells of the same tissue type as the test cell, and determining the approximate amount of immunocomplex formation by the antibody and the proteins of the test cell, wherein a statistically significant difference in the amount of the immunocomplex formed with the proteins of a test cell as compared to a normal cell of the same tissue type is an indication that the test cell is cancerous or pre-cancerous.

The method for producing polyclonal and/or monoclonal antibodies which specifically bind to polypeptides useful in the present invention is known to those of skill in the art and may be found in, for example, Dymecki, et al., (J. Biol. Chem. 267:4815, 1992); Boersma & Van Leeuwen, (J. Neurosci. Methods 51:317, 1994); Green, et al., (Cell 28:477, 1982); and Arnheiter, et al., (Nature 294:278, 1981).

Another such method includes the steps of: providing an antibody specific for the gene product of a marker nucleic acid sequence, the gene product being present in cancerous tissue of a given tissue type at a level more or less than the level of the gene product in non-cancerous tissue of the same tissue type; obtaining from a patient a first sample of tissue of the given tissue type, which sample potentially includes cancerous cells; providing a second sample of tissue of the same tissue type (which may be from the same patient or from a normal control, e.g. another individual or cultured cells), this second sample containing normal cells and essentially no cancerous cells; contacting the antibody with protein (which may be partially purified, in lysed but unfractionated cells, or *in situ*) of the first and second samples under conditions permitting immunocomplex formation between the antibody and the marker nucleic acid sequence product present in the samples; and comparing (a) the amount of immunocomplex formation in the first sample, with (b) the amount of immunocomplex formation in the second sample, wherein a statistically significant difference in the amount of immunocomplex formation in the first sample less as compared to the amount of immunocomplex formation in the second sample is indicative of the presence of cancerous cells in the first sample of tissue.

The subject invention further provides a method of determining whether a cell sample obtained from a subject possesses an abnormal amount of marker polypeptide which comprises (a) obtaining a cell sample from the subject, (b) quantitatively determining the amount of the marker polypeptide in the sample so obtained, and (c) comparing the amount of the marker polypeptide so determined with a known standard, so as to thereby determine whether the cell sample obtained from the subject possesses an abnormal amount of the marker polypeptide. Such marker polypeptides may be detected by immunohistochemical assays, dot-blot assays, ELISA, and the like.

Immunoassays are commonly used to quantitate the levels of proteins in cell samples, and many other immunoassay techniques are known in the art. The invention is not limited to a particular assay procedure, and therefore, is intended to include both homogeneous and heterogeneous procedures. Exemplary immunoassays which may be conducted according to the invention include fluorescence polarization immunoassay (FPIA), fluorescence immunoassay (FIA), enzyme immunoassay (EIA), nephelometric inhibition immunoassay (NIA), enzyme-linked immunosorbent assay (ELISA), and radioimmunoassay (RIA). An indicator moiety, or label group, may be attached to the subject antibodies and is selected so as to meet the needs of various uses of the method which are often dictated by the availability of assay equipment and compatible immunoassay procedures. General techniques to be used in performing the various immunoassays noted above are known to those of ordinary skill in the art.

In another embodiment, the level of the encoded product, or alternatively the level of the polypeptide, in a biological fluid (e.g., blood or urine) of a patient may be determined as a way of monitoring the level of expression of the marker nucleic acid sequence in cells of that patient. Such a method would include the steps of obtaining a sample of a biological fluid from the patient, contacting the sample (or proteins from the sample) with an antibody specific for an encoded marker polypeptide, and determining the amount of immune complex formation by the antibody, with the amount of immune complex formation being indicative of the level of the marker encoded product in the sample. This determination is particularly instructive when compared to the amount of immune complex formation by the same antibody in a control sample taken from a normal individual or in one or more samples previously or subsequently obtained from the same person.

In another embodiment, the method may be used to determine the amount of marker polypeptide present in a cell, which in turn may be correlated with progression of a hyperproliferative disorder. The level of the marker polypeptide may be used predictively to evaluate whether a sample of cells contains cells which are, or are predisposed towards becoming, transformed cells. Moreover, the subject method may be used to assess the phenotype of cells which are known to be transformed, the phenotyping results being useful in planning a particular therapeutic regimen. For example, very high levels of the marker polypeptide in sample cells is a powerful diagnostic and prognostic marker for a cancer. The observation of marker polypeptide levels may be utilized in decisions regarding, for example, the use of more aggressive therapies.

As set out above, one aspect of the present invention relates to diagnostic assays for determining, in the context of cells isolated from a patient, if the level of a marker polypeptide is significantly reduced in the sample cells. The term "significantly reduced" refers to a cell phenotype wherein the cell possesses a reduced cellular amount of the marker polypeptide relative to a normal cell of similar tissue origin. The assay evaluates the level of marker polypeptide in the test cells, and may compare the measured level with marker polypeptide detected in at least one control cell, for example, a normal cell and/or a transformed cell of known phenotype.

Another aspect of the subject invention is the ability to quantitate the level of marker polypeptide as determined by the number of cells associated with a normal or abnormal marker polypeptide level. The number of cells with a particular marker polypeptide phenotype may then be correlated with patient prognosis. In one embodiment of the invention, the marker polypeptide phenotype of a lesion is determined as a percentage of cells in a biopsy which are found to have abnormally high/low levels of the marker polypeptide. Such expression may be detected by immunohistochemical assays, dot-blot assays, ELISA, and the like.

Where tissue samples are employed, immunohistochemical staining may be used to determine the number of cells having the marker polypeptide phenotype. For such staining, a multiblock of tissue may be taken from the biopsy or other tissue sample and subjected to proteolytic hydrolysis, employing such agents as protease K or pepsin. In certain embodiments, it may be desirable to isolate a nuclear fraction from the sample cells and detect the level of the marker polypeptide in the nuclear fraction.

The tissue samples are fixed by treatment with a reagent such as formalin, glutaraldehyde, methanol, or the like. The samples are then incubated with an antibody (e.g., a monoclonal antibody) with binding specificity for the marker polypeptides. This antibody may be conjugated to a label for subsequent detection of binding. Samples are incubated for a time sufficient for formation of the immunocomplexes. Binding of the antibody is then detected by virtue of a label conjugated to this antibody. Where the antibody is unlabeled, a second labeled antibody may be employed, for example, which is specific for the isotype of the anti-marker polypeptide antibody. Examples of labels which may be employed include radionuclides, fluorescers, chemiluminescers, enzymes, and the like.

Where enzymes are employed, the substrate for the enzyme may be added to the samples to provide a colored or fluorescent product. Examples of suitable enzymes for use in conjugates include horseradish peroxidase, alkaline phosphatase, malate dehydrogenase, and the like. Where not commercially available, such antibody-enzyme conjugates are readily produced by techniques known to those skilled in the art.

In one embodiment, the assay is performed as a dot blot assay. The dot blot assay finds particular application where tissue samples are employed as it allows determination of the average amount of the marker polypeptide associated with a single cell by correlating the amount of marker polypeptide in a cell-free extract produced from a predetermined number of cells.

It is well established in the cancer literature that tumor cells of the same type (e.g., lung and/or colon tumor cells) may not show uniformly increased expression of individual oncogenes or uniformly decreased expression of individual tumor suppressor genes. There may also be varying levels of expression of a given marker gene even between cells of a given type of cancer, further emphasizing the need for reliance on a battery of tests rather than a single test. Accordingly, in one aspect, the invention provides for a battery of tests utilizing a number of probes of the invention, in order to improve the reliability and/or accuracy of the diagnostic test.

In one embodiment, the present invention also provides a method wherein nucleic acid probes are immobilized on a DNA chip in an organized array. Oligonucleotides may be bound to a solid support by a variety of processes, including lithography. For example, a chip may hold up to 250,000 oligonucleotides. These nucleic acid probes comprise a nucleotide sequence, for example, at least about 12, 15, 25, or 40 nucleotides in length, and up to all or nearly all of a sequence which is complementary to a portion of the coding sequence of a marker nucleic acid sequence and is differentially expressed in tumor cells. The present invention provides significant advantages over the available tests for various cancers, because it increases the reliability of the test by providing an array of nucleic acid markers on a single chip.

The method includes obtaining a biopsy, which is optionally fractionated by cryostat sectioning to enrich tumor cells. The DNA or RNA is then extracted, amplified, and analyzed with a DNA chip to determine the presence of absence of the marker nucleic acid sequences.

In one embodiment, the nucleic acid probes are spotted onto a substrate in a two-dimensional matrix or array. Samples of nucleic acids may be labeled and then hybridized to the probes. Double-stranded nucleic acids, comprising the labeled sample nucleic acids bound to probe nucleic acids, may be detected once the unbound portion of the sample is washed away.

The probe nucleic acids may be spotted on substrates including glass, nitrocellulose, etc. The probes can be bound to the substrate by either covalent bonds or by non-specific interactions, such as hydrophobic interactions. The sample nucleic acids can be labeled using radioactive labels, fluorophores, chromophores, etc.

In yet another embodiment, the invention contemplates using a panel of antibodies which are generated against the marker polypeptides of this invention. Such a panel of antibodies may be used as a reliable diagnostic probe for cancer. The assay of the present invention comprises contacting a biopsy sample containing cells, for example, lung cells, with a panel of antibodies to one or more of the encoded products to determine the presence or absence of the marker polypeptides.

The diagnostic methods of the subject invention may also be employed as follow-up to treatment, for example, quantitation of the level of marker polypeptides may be indicative of the effectiveness of current or previously employed cancer therapies as well as the effect of these therapies upon patient prognosis.

In addition, the marker nucleic acids or marker polypeptides may be utilized as part of a diagnostic panel for initial detection, follow-up screening, detection of reoccurrence, and post-treatment monitoring for chemotherapy or surgical treatment.

Accordingly, the present invention makes available diagnostic assays and reagents for detecting gain and/or loss of marker polypeptides from a cell in order to aid in the diagnosis and phenotyping of proliferative disorders arising from, for example, tumorigenic transformation of cells.

The diagnostic assays described above may be adapted to be used as prognostic assays, as well. Such an application takes advantage of the sensitivity of the assays of the invention to events which take place at characteristic stages in the progression of a tumor. For example, a given marker gene may be up- or down-regulated at a very early stage, perhaps before the cell is irreversibly committed to developing into a malignancy, while another marker gene may be characteristically up- or down-regulated only at a much later stage. Such a method could involve the steps of contacting the mRNA of a test cell with a nucleic acid probe derived from a given marker nucleic acid which is expressed at different characteristic levels in cancerous or precancerous cells at different stages of tumor progression, and determining the approximate amount of hybridization of the probe to the mRNA of the cell, such amount being an indication of the level of expression of the gene in the cell, and thus an indication of the stage of tumor progression of the cell; alternatively, the assay may be carried out with an antibody specific for the gene product of the given marker nucleic acid, contacted with the proteins of the test cell. A battery of such tests will disclose not only the existence and location of a tumor, but also will allow the clinician to select the mode of treatment most appropriate for the tumor, and to predict the likelihood of success of that treatment.

The methods of the invention may also be used to follow the clinical course of a tumor. For example, the assay of the invention may be applied to a tissue sample from a patient; following treatment of the patient for the cancer, another tissue sample is taken and the test repeated. Successful treatment will result in either removal of all cells which demonstrate differential expression characteristic of the cancerous or precancerous cells, or a substantial increase in expression of the gene in those cells, perhaps approaching or even surpassing normal levels.

In yet another embodiment, the invention provides methods for determining whether a subject is at risk for developing a disease, such as a predisposition to develop cancer, associated with aberrant activity of a polypeptide, wherein the aberrant activity of the polypeptide is characterized by detecting the presence or absence of a genetic lesion characterized by at least one of (a) an alteration affecting the integrity of a gene encoding a marker polypeptides, or (b) the mis-expression of the encoding nucleic acid. To illustrate, such genetic lesions may be detected by ascertaining the existence of at least one of (i) a deletion of one or more nucleotides from the nucleic acid sequence, (ii) an addition of one or more nucleotides to the nucleic acid sequence, (iii) a substitution of one or more nucleotides of the nucleic acid sequence, (iv) a gross chromosomal rearrangement of the nucleic acid sequence, (v) a gross alteration in the level of a messenger RNA transcript of the nucleic acid sequence, (vi) aberrant modification of the nucleic acid sequence, such as of the methylation pattern of the genomic DNA, (vii) the presence of a non-wild type splicing pattern of a messenger RNA transcript of the gene, (viii) a non-wild type level of the marker polypeptide, (ix) allelic loss of the gene, and/or (x) inappropriate post-translational modification of the marker polypeptide.

The present invention provides assay techniques for detecting lesions in the encoding nucleic acid sequence. These methods include, but are not limited to, methods involving sequence analysis, Southern blot hybridization, restriction enzyme site mapping, and methods involving detection of absence of nucleotide pairing between the nucleic acid to be analyzed and a probe.

Specific diseases or disorders, for example, genetic diseases or disorders, are associated with specific allelic variants of polymorphic regions of certain genes, which do not necessarily encode a mutated protein. Thus, the presence of a specific allelic variant of a polymorphic region of a gene in a subject may render the subject susceptible to developing a specific disease or disorder. Polymorphic regions in genes, may be identified, by determining the nucleotide sequence of genes in populations of individuals. If a polymorphic region is identified, then the link with a specific disease may be determined by studying specific populations of individuals, for example, individuals which developed a specific disease, such as cancer. A polymorphic region may be located in any region of a gene, for example, exons, in coding or non-coding regions of exons, introns, and promoter region.

In an exemplary embodiment, there is provided a nucleic acid composition comprising a nucleic acid probe including a region of nucleotide sequence which is capable of hybridizing to a sense or antisense sequence of a gene or naturally occurring mutants thereof, or 5' or 3' flanking sequences or intronic sequences naturally associated with the subject genes or naturally occurring mutants thereof. The nucleic acid of a cell is rendered accessible for hybridization, the probe is contacted with the nucleic acid of the sample, and the hybridization of the probe to the sample nucleic acid is detected. Such techniques may be used to detect lesions or allelic variants at either the genomic or mRNA level, including deletions, substitutions, etc., as well as to determine mRNA transcript levels.

An example of a detection method is allele specific hybridization using probes overlapping the mutation or polymorphic site and having about 5, 10, 20, 25, or 30 nucleotides around the mutation or polymorphic region. In one embodiment of the invention, several probes capable of hybridizing specifically to allelic variants are attached to a solid phase support, for example, a "chip." Mutation detection analysis using these chips comprising oligonucleotides, also termed "DNA probe arrays" is described, for example, by Cronin, et al., (Human Mutation 7:244, 1996). In one embodiment, a chip may comprise all the allelic variants of at least one polymorphic region of a gene. The solid phase support is then contacted with a test nucleic acid and hybridization to the specific probes is detected. Accordingly, the identity of numerous allelic variants of one or more genes may be identified in a simple hybridization experiment.

In certain embodiments, detection of the lesion comprises utilizing the probe/primer in a polymerase chain reaction (PCR) (see, e.g., U.S. Patent Nos. 4,683,195 and 4,683,202), such as anchor PCR or RACE PCR, or, alternatively, in a ligase chain reaction (LCR) (see, e.g., Landegran, et al., Science 241:1077-1080, 1988; Nakazaw, et al., Proc. Natl. Acad. Sci. USA 91:360-364, 1994), the latter of which can be particularly useful for detecting point mutations in the gene (see, e.g., Abravaya, et al., Nuc. Acid Res. 23:675-682, 1995). In an illustrative embodiment, the method includes the steps of (i) collecting a sample of cells from a patient, (ii) isolating nucleic acid (e.g., genomic, mRNA, or both) from the cells of the sample, (iii) contacting the nucleic acid sample with one or more primers which specifically hybridize to a nucleic acid sequence under conditions such that hybridization and amplification of the nucleic acid (if present) occurs, and (iv) detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

The invention thus, also encompasses methods of screening for agents which inhibit or enhance the expression of the nucleic acid markers *in vitro,* comprising exposing a cell or tissue in which the marker nucleic acid mRNA is detectable in cultured cells to an agent in order to determine whether the agent is capable of inhibiting or enhancing production of the mRNA; and determining the level of mRNA in the exposed cells or tissue, wherein a decrease in the level of the mRNA after exposure of the cell line to the agent is indicative of inhibition of the marker nucleic acid mRNA production and an increase in mRNA levels is indicative of enhancement of maker mRNA production.

Alternatively, the screening method may include *in vitro* screening of a cell or tissue in which marker protein is detectable in cultured cells to an agent suspected of inhibiting or enhancing production of the marker protein; and determining the level of the marker protein in the cells or tissue, wherein a decrease in the level of marker protein after exposure of the cells or tissue to the agent is indicative of inhibition of marker protein production and an increase on the level of marker protein is indicative of enhancement of marker protein production.

The invention also encompasses *in vivo* methods of screening for agents which inhibit or enhance expression of the marker nucleic acids, comprising exposing a subject having tumor cells in which marker mRNA or protein is detectable to an agent suspected of inhibiting or enhancing production of marker mRNA or protein; and determining the level of marker mRNA or protein in tumor cells of the exposed mammal. A decrease in the level of marker mRNA or protein after exposure of the subject to the agent is indicative of inhibition of marker nucleic acid expression and an increase in the level of marker RNA or protein is indicative of enhancement of marker nucleic acid expression.

Accordingly, the invention provides a method comprising incubating a cell expressing the marker nucleic acids with a test compound and measuring the mRNA or protein level. The invention further provides a method for quantitatively determining the level of expression of the marker nucleic acids in a cell population, and a method for determining whether an agent is capable of increasing or decreasing the level of expression of the marker nucleic acids in a cell population. The method for determining whether an agent is capable of increasing or decreasing the level of expression of the marker nucleic acids in a cell population comprises the steps of (a) preparing cell extracts from control and agent-treated cell populations, (b) isolating the marker polypeptides from the cell extracts, and (c) quantifying (e.g., in parallel) the amount of an immunocomplex formed between the marker polypeptide and an antibody specific to said polypeptide. The marker polypeptides of this invention may also be quantified by assaying for its bioactivity. Agents that induce an increase in the marker nucleic acid expression may be identified by their ability to increase the amount of immunocomplex formed in the treated cell as compared with the amount of the immunocomplex formed in the control cell. In a similar manner, agents that decrease expression of the marker nucleic acid may be identified by their ability to decrease the amount of the immunocomplex formed in the treated cell extract as compared to the control cell.

### Predictive Assays

Laboratory-based assays, which can predict clinical benefit from a given anti-cancer agent, will greatly enhance the clinical management of patients with cancer. In order to assess this effect, a biomarker associated with the anti-cancer agent may be analyzed in a biological sample (e.g., tumor sample, plasma) before, during, and following treatment.

Another approach to monitor treatment is an evaluation of serum proteomic spectra. Specifically, plasma samples may be subjected to mass spectroscopy (e.g., surface-enhanced laser desorption and ionization) and a proteomic spectra may be generated for each patient. A set of spectra, derived from analysis of plasma from patients before and during treatment, may be analyzed by an iterative searching algorithm, which can identify a proteomic pattern that completely discriminates the treated samples from the untreated samples. The resulting pattern may then be used to predict the clinical benefit following treatment.

Global gene expression profiling of biological samples (e.g., tumor biopsy samples, blood samples) and bioinformatics-driven pattern identification may be utilized to predict clinical benefit and sensitivity, as well as development of resistance to an anti-cancer agent. For example, RNA isolated from cells derived from whole blood from patients before and during treatment may be used to generate blood cell gene expression profiles utilizing Affymetrix GeneChip technology and algorithms. These gene expression profiles may then predict the clinical benefit from treatment with a particular anti-cancer agent.

Analysis of the biochemical composition of urine by 1D ¹H-NMR (Nuclear Magnetic Resonance) may also be utilized as a predictive assay. Pattern recognition techniques may be used to evaluate the metabolic response to treatment with an anti-cancer agent and to correlate this response with clinical endpoints. The biochemical or endogenous metabolites excreted in urine have been well-characterized by proton NMR for normal subjects (Zuppi, et al., Clin Chim Acta 265:85-97, 1997). These metabolites (approximately 30-40) represent the by-products of the major metabolic pathways, such as the citric acid and urea cycles. Drug-, disease-, and genetic-stimuli have been shown to produce metabolic-specific changes in baseline urine profiles that are indicative of the timeline and magnitude of the metabolic response to the stimuli. These analyses are multi-variant and therefore use pattern recognition techniques to improve data interpretation. Urinary metabolic profiles may be correlated with clinical endpoints to determine the clinical benefit. *Kits*

The invention further provides kits for determining the expression level of genes characteristic of small molecule efficacy. The kits may be useful for identifying subjects that are predisposed to developing cancer or who have cancer, as well as for identifying and validating therapeutics for cancer. In one embodiment, the kit comprises a computer readable medium on which is stored one or more gene expression profile of diseased cells of cancer, or at least values representing levels of expression of one or more genes characteristic of small molecule efficacy in a diseased cell. The computer readable medium can also comprise gene expression profiles of counterpart normal cells, diseased cells treated with a drug, and any other gene expression profile described herein. The kit can comprise expression profile analysis software capable of being loaded into the memory of a computer system.

A kit can comprise a microarray comprising probes of genes characteristic of small molecule efficacy. A kit can comprise one or more probes or primers for detecting the expression level of one or more genes characteristic of small molecule efficacy and/or a solid support on which probes attached and which can be used for detecting expression of one or more genes characteristic of small molecule efficacy in a sample. A kit may further comprise nucleic acid controls, buffers, and instructions for use.

Other kits provide compositions for treating cancer. For example, a kit can also comprise one or more nucleic acids corresponding to one or more genes characteristic of small molecule efficacy (e.g., for use in treating a patient having cancer). The nucleic acids can be included in a plasmid or a vector (e.g., a viral vector). Other kits comprise a polypeptide encoded by a gene characteristic of cancer or an antibody to a polypeptide. Yet other kits comprise compounds identified herein as agonists or antagonists of genes characteristic of small molecule efficacy. The compositions may be pharmaceutical compositions comprising a pharmaceutically acceptable excipient.

### EXAMPLES

### Example 1. Gene Expression Profiling Protocol

### A. Blood Source

Human blood was obtained from patients treated with the drug sorafenib prior to treatment. Patients were divided into two groups, those that had a best response, as determined by WHO criteria, of progressive disease ("progressors") and those that had a best response of stable disease or better ("non-progressors").

### B. Total RNA Isolation From Human Whole Blood

This method utilizes the Qiagen QIAamp RNA Blood Mini kit: Version 01/99: "QIAamp RNA Mini Protocol for Isolation of Total Cellular RNA from Whole Human Blood" (http://www.giagen.com/literature/Handbooks/PDF/DNA RNA isolation clinical/INT/QA RNA Blood Mini/garnab blood.pdf)

**Protocol**
1. Remove tubes of whole blood from -80°C freezer; place tubes in ice.
2. Partially thaw blood on ice.
   ■ Leave tubes submerged in ice up to tube cap.
   ■ Thaw until the blood is an icy slurry which will move freely when the tube is inverted.
3. Add 1 volume of whole blood to 3 volumes of ice-cold Buffer EL (modified Step 1 of protocol).
   ■ Place 50 ml conical tube on ice with 2 volumes of Buffer EL.
   ■ Pour partially thawed whole blood into 50 ml conical tube containing ice-cold Buffer EL.
   ■ Use remaining **1** volume of ice-cold Buffer EL to rinse residual blood from first tube.
4. Invert the 50 ml conical tube gently several times; return tube to ice.
5. Incubate tubes on ice for 10 minutes, gently inverting tube several times during incubation.
6. Spin tubes for 10' at 1000 RPM in tabletop centrifuge, 4°C.
7. Gently decant supernatant to biohazardous waste; place 50 ml conical tube on ice.
8. Gently pipet 1 volume of ice-cold Buffer EL down side of conical tube.
9. Gently swirl tube to resuspend lymphocyte cell pellet; pour into 15 ml conical tube which has been placed on ice.
10. Use an additional 0.5 volume of ice-cold Buffer EL to rinse the 50 ml conical tube; combine with first wash in 15 ml conical tube.
11. Spin tubes for 5' at 1000 RPM in tabletop centrifuge, 4°C.
12. Carefully remove supernatant using narrow pipet tip and vacuum source, taking care to not disturb cell pellet.
13. Add 580 ul of Buffer RLT (containing B-ME) to cell pellet; vortex vigorously to solubilize cell pellet.
14. Either (a) freeze cell lysate at -80°C, or (b) continue with Step 7 of Qiagen protocol.
15. Include optional on-column DNase digestion step using Qiagen RNase-Free DNase Set; DNase digest for 30' at room temperature.
16. Modify DNase digestion protocol by using 500 ul (instead of 350 ul) Buffer RW1 to wash column both before and after DNase digestion.
17. Include option 12a.
18. Elute RNA with RNase-Free water provided; add 44 ul directly onto filter, let sit for 1 minute, and then centrifuge for 1 minute at ≥10,000rpm. Do not repeat elution.
19. Store tubes of eluted blood RNA at -80 C.

### C. Affymetrix GeneChip Microarray Method

First and second strand cDNA synthesis is performed using 500 ng to 5 ug RNA using the Superscript Choice System (Invirogen; Carlsbad, CA) as described by Affymetrix protocol. Briefly, 100pmol T7 d(T)₂₄ primer (Invitrogen, CA) was added to the RNA, incubated at 70°C for 10 minutes, and quenched on ice. The mix was then incubated at 42°C for 2 minutes in first strand cDNA buffer, 10 mM DTT, and 1mM dNTPs. Then, 200U SSII reverse transcriptase were added and further incubated for 1 hour at 42°C. Each tube was then incubated for 2 hours at 16°C after adding second strand buffer, 0.2 mM dNTPs, 10U E. Coli DNA ligase, 40U DNA polymerase I, and 2U RNase H. After the 2 hours, 10U T4 DNA polymerase was added and the reaction was incubated for another 5 minutes at 16°C. The reaction was stopped by addition of 0.5 M EDTA and the cDNA was purified using phenol:chloroform:isoamyl alcohol extraction, followed by ethanol precipitation with 7.5 M NH₄OAc The pellet was resuspended in DEPC H₂O.

cRNA for array analyses was generated by *in vitro* transcription of the cDNA using the BioArray HighYield Transcription Kit (Enzo Diagnostics, NY) as described by the manufacturer. Briefly, template cDNA was mixed with HY buffer, Biotin NTPs, DTT, RNase inhibitor, and T7 RNA polymerase. Tubes were incubated at 37°C for 5 hours. The cRNA was purified using an RNeasy clean-up procedure and measured by UV spectroscopy. Up to 15 µg cRNA was fragmented at 94°C for 35min in .2M Tris-acetate, 0.5M KOAc, and 0.15 M MgOAc. For array hybridization, cocktails were made of fragmented cRNA, 50 pM control oligo B2, control cRNA spikes from Affymetrix, 30 µg herring sperm DNA, 150 µg BSA, and Hyb Buffer (100mM MES, 1 M Na⁺, 20mM EDTA, .01% Tween20).

HG-U133 Plus 2.0 arrays (containing over 60,000 probe sets representing approximately 50,000 RNA transcripts) from Affymetrix were pre-hybridized with Hybridization Buffer at 45°C for 10 minutes rotating 60rpm in the GeneChip Hybridization Oven 640 and then hybridized overnight with a portion of the sample hybridization cocktail.

The chips were then put through a series of washes with 6X SSPE, .01 % Tween 20, 0.005% Antifoam, and 100mM MES. They were stained with 6ug Phycoerythrin-Streptavidin (Molecular Probes, CA) in 100mM MES and 1.2mg BSA. The chips were scanned by the GeneChip Scanner 3000 (GCS3000) at 488nm and analyzed using MicroArray Suite 5.0 software from Affymetrix. The software digitally converted the intensity of light given off by the array into a numeric value indicative of levels of gene expression.

### D. Data analysis

The purpose is to generate sets of markers to distinguish between progressors and non-progressors. Marker Set One (Table 1) represents a set of probe sets that is an optimum set for the prediction of whether or not a patient is a progressor or a non-progressor using a support vector machine. The optimal set is determined to be the one that shows the greatest prediction accuracy as well as a relatively small number of genes. This marker set was derived using the following method:
1. The data was imported into Spotfire.
2. A Treatment Comparison between progressors and non-progressors was performed.
3. The following criteria were used to select the probe sets:
   a. The data showed that the probe sets were all not "Absent," as determined by the Affymetrix¹ Microarray Suite software v. 5.0 for greater than 85% of the patients in either group.
      ¹ Affymetrix, Inc., Santa Clara, CA
   b. All probe sets not meeting these criteria were eliminated from further analysis.
4. The remaining data was used in a selection process using the WEKA² software package and an algorithm called a Support Vector Machine. The software ranked the probe sets as to their relevance for predicting best response in the patients.
   ² described in Data Mining: Practical Machine Learning Tools and Techniques with Java Implementations. Ian H. Witten, Eibe Frank. Morgan Kaufmann, October 1999. ISBN 1-55860-552-5
5. Numerous models were generated using subsets of the top-ranked probe sets in order to determine a small subset that yielded an estimated accuracy of 100%, as determined by cross-validation, when predicting whether or not a patient would be a progressor or non-progressor.
6. The probe sets, along with annotation³, that yielded the best model meeting the criteria above are listed in Table 1.
   ³ From Affymetrix web site address (file dated 16Sep2005) http://www.affymetrix.com/support/technical/byproduct.affx?product=hg-u133-plus

**Table 1**

| SEQ ID NO: | ProbeSet | UniGene ID | Gene Symbol | Gene Title |
|---|---|---|---|---|
| 1 | 243570_at | Hs.282700 | SPCS2 | Signal peptidase complex subunit 2 homolog (S. cerevisiae) |
| 2 | 218287_s_at | Hs.22867 | EIF2C1 | eukaryotic translation initiation factor 2C, 1 |
| 3 | 213957_s_at | Hs.413045 | CAP350 | centrosome-associated protein 350 |
| 4 | 225469_at | Hs.209151 | LOC144363 | hypothetical protein LOC144363 |
| 5 | 226154_at | Hs.505231 /// Hs.550499 | DNM1L /// CGI-04 | Dynamin 1-like /// CGI-04 protein |
| 6 | 53720_at | Hd.175120 | FLJ11286 | hypothetical protein FLJ11286 |
| 7 | 202509_s_at | Hs.525607 | TNFAIP2 | tumor necrosis factor, alpha-induced protein 2 |
| 8 | 241408_at | Hs.26410 | FLJ34443 | hypothetical protein |
| 9 | 200732_s_at | Hs.227777 | PTP4A1 | protein tyrosine phosphatase type IVA, member 1 |
| 10 | 228329_at | Hs.4204 | --- | CDNA FLJ30779 fis, clone FEBRA2000815 |
| 11 | 225028_at | Hs.355559 | LOC550643 | hypothetical protein LOC550643 |
| 12 | 214440_at | Hs.155956 | NAT1 | N-acetyltransferase 1 (arylamine N-acetyltransferase) |
| 13 | 203405_at | Hs.473838 | DSCR2 | Down syndrome critical region gene 2 |
| 14 | 213379_at | Hs.144304 | COQ2 | coenzyme Q2 homolog, prenyltransferase (yeast) |
| 15 | 214370_at | Hs.416073 | S100A8 | S100 calcium binding protein A8 (calgranulin A) |
| 16 | 221190_s_at | Hs.529006 | C18orf8 | chromosome 18 open reading_frame 8 |
| 17 | 210357_s_at | Hs.433337 | SMOX | spermine oxidase |
| 18 | 32209_at | Hs.25723 | MTVR1 | Mouse Mammary Turmor Virus Receptor homolog 1 |

## Claims

1. A method to predict the response of a hepatocellular carcinoma patient to the multi-kinase inhibitor sorafenib, comprising the steps of:
(a) determining the mRNA expression profile of genes in a blood sample taken from the patient prior to treatment with the multi-kinase inhibitor, and
(b) comparing the mRNA expression profile of genes in the blood sample with mRNA expression profiles of said genes obtained from hepatocellular carcinoma patients having progressive disease and patients having stable disease or better after sorafenib treatment, wherein said mRNA expression profiles of hepatocellular carcinoma patients having progressive disease and patients having stable disease or better were obtained prior to sorafenib treatment;
wherein the genes comprise the group consisting of genes 1-18 in the following table:
| SEQ ID NO: | ProbeSet | UniGene ID | Gene Symbol | Gene Title |
|---|---|---|---|---|
| 1 | 243570 at | Hs.282700 | SPCS2 | Signal peptidase complex subunit 2 homolog (S. cerevisiae) |
| 2 | 218287_s_at | Hs.22867 | EIF2C1 | eukaryotic translation initiation factor 2C, 1 |
| 3 | 213957_s_at | Hs.413045 | CAP350 | centrosome-associated protein 350 |
| 4 | 225469 at | Hs.209151 | LOC144363 | hypothetical protein LOC144363 |
| 5 | 226154_at | Hs.505231 /// Hs.550499 | DNM1L /// CGI-04 | Dynamin 1-like /// CGI-04 protein |
| | | | | |
|---|---|---|---|---|
| 6 | 53720 at | Hs.175120 | FLJ11286 | hypothetical protein FLJ11286 |
| 7 | 202509 s at | Hs.525607 | TNFAIP2 | tumor necrosis factor, alpha-induced protein 2 |
| 8 | 241408 at | Hs.26410 | FLJ34443 | hypothetical protein FLJ34443 |
| 9 | 200732_s_at | Hs.227777 | PTP4A1 | protein tyrosine phosphatase type IVA, member 1 |
| 10 | 228329 at | Hs.4204 | - | CDNA FLJ30779 fis, clone FEBRA2000815 |
| 11 | 225028 at | Hs.355559 | LOC550643 | hypothetical protein LOC550643 |
| 12 | 214440_at | Hs.155956 | NAT1 | N-acetyltransferase 1 (arylamine N-acetyltransferase) |
| 13 | 203405 at | Hs.473838 | DSCR2 | Down syndrome critical region gene 2 |
| 14 | 213379 at | Hs.144304 | COQ2 | coenzyme Q2 homolog, prenyltransferase (yeast) |
| 15 | 214370_at | Hs.416073 | S100A8 | S100 calcium binding protein A8 (calgranulin A) |
| 16 | 221190_s_at | Hs.529006 | C18orf8 | chromosome 18 open reading frame 8 |
| 17 | 210357 s at | Hs.433337 | SMOX | spermine oxidase |
| 18 | 32209 at | Hs.25723 | MTVR1 | Mouse Mammary Turmor Virus Receptor homolog 1 |

## Patentansprüche

1. Verfahren zur Vorhersage des Ansprechens eines Patienten mit hepatozellulärem Karzinom auf den Multi-Kinase-Inhibitor Sorafenib, mit den folgenden Schritten:
(a) Bestimmen des mRNA-Expressionsprofils von Genen in einer dem Patienten vor der Behandlung mit dem Multi-Kinase-Inhibitor entnommenen Blutprobe und
(b) Vergleichen des mRNA-Expressionsprofils von Genen in der Blutprobe mit von Patienten mit fortgeschrittenem hepatozellulärem Karzinom und Patienten mit stabilem hepatozellulärem Karzinom oder besser gewonnenen mRNA-Expressionsprofilen der Gene nach Behandlung mit Sorafenib, wobei die mRNA-Expressionsprofile von Patienten mit fortgeschrittenem hepatozellulärem Karzinom und Patienten mit stabilem hepatozellulärem Karzinom oder besser vor Behandlung mit Sorafenib gewonnen wurden;
wobei die Gene die aus Genen 1-18 in der folgenden Tabelle bestehende Gruppe umfassen:
| SEQ ID NO: | Probeset | UniGene ID | Gensymbol | Genname |
|---|---|---|---|---|
| 1 | 243570_at | Hs.282700 | SPCS2 | Homolog der Signalpeptidase-Komplex-Untereinheit 2 (S. Cerevisiae) |
| 2 | 218287_s_at | Hs.22867 | EIF2C1 | Eukaryontischer Translationsinitiationsfaktor 2C, 1 |
| 3 | 213957_s_at | Hs. 413045 | CAP350 | Centrosom-assoziiertes Protein 350 |
| 4 | 225469_at | Hs.209151 | LOC144363 | Hypothetisches Protein LOC144363 |
| 5 | 226154_at | Hs.505231 /// Hs.550499 | DNM1L /// CGI-04 | Dynamin-1-like- /// CGI-04-Protein |
| 6 | 53720_at | Hs.175120 | FLJ11286 | Hypothetisches Protein FLJ11286 |
| 7 | 202509_s_at | Hs.525607 | TNFAIP2 | Tumornekrosefaktor, alpha-induziertes Protein 2 |
| 8 | 241408-at | Hs.26410 | FLJ34443 | Hypothetisches Protein FLJ34443 |
| 9 | 200732_s_at | Hs.227777 | PTP4A1 | Protein-Tyrosinphosphatase Typ IVA, Mitgleid 1 |
| 10 | 228329_at | Hs.4204 | --- | CDNA FLJ30779 fis, Klon FEBRA2000815 |
| 11 | 225028_at | Hs.355559 | LOC550643 | Hypothetisches Protein LOC550643 |
| 12 | 214440_at | Hs.155956 | NAT1 | N-Acetyltransferase 1 (Arylamin-N-Acetyltransferase) |
| 13 | 203405_at | Hs.473838 | DSCR2 | Down-Syndrom-kritischer-Bereich-Gen 2 |
| 14 | 213379_at | Hs.144304 | COQ2 | Coenzym-Q2-Homolog, Prenyltransferase (Hefe) |
| 15 | 214370_at | Hs.416073 | S100A8 | S100 Calcium-Bindungsprotein A8 (Calgranulin A) |
| 16 | 221190_s_at | Hs.529006 | C18orf8 | Chromosom 18, offenes Leseraster 8 |
| 17 | 210357 s at | Hs.433337 | SMOX | Spermin-Oxidase |
| 18 | 32209 at | Hs.25723 | MTVR1 | MMTV-Rezeptor-Homolog 1 |

## Revendications

1. Procédé de prédiction de la réaction d'un patient à carcinome hépatocellulaire à l'inhibiteur de multi-kinase sorafénib, comprenant les stades dans lesquels :
(a) on détermine le profil d'expression de l'ARNm de gènes dans un échantillon de sang pris chez le patient avant un traitement par l'inhibiteur de multi-kinase, et
(b) on compare le profil d'expression de l'ARNm de gènes de l'échantillon de sang à des profils d'expression de l'ARNm de ces gènes obtenus chez des patients à carcinome hépatocellulaire ayant une maladie en progression et chez des patients ayant une maladie stable ou une amélioration après un traitement par le sorafénib, les profils d'expression de l'ARNm de patients à carcinome hépatocellulaire ayant une maladie en progression et de patients ayant une maladie stable ou une amélioration étant obtenus avant un traitement par le sorafénib ;
dans lequel les gènes comprennent le groupe consistant en les gènes 1 à 18 du tableau suivant :
| **Identification de séquence N°** | **Groupe de sonde** | **ID d'unigène** | **Symbole de Gène** | **Titre de Gène** |
|---|---|---|---|---|
| 1 | 243570_at | Hs.282700 | SPCS2 | Homologue de sous unité 2 de complexe de signal peptidase (S. cerivisae) |
| 2 | 218287_s_at | Hs.22867 | EIF2C1 | Facteur d'initiation de traduction eucaryotique 2C, 1 |
| 3 | 213957_s_at | Hs.413045 | CAP350 | Protéine associée au centrosome 350 |
| 4 | 225469_at | Hs.209151 | LOC144363 | Protéine hypothétique LOC144363 |
| 5 | 226154_at | Hs.505231 /// Hs.550499 | DNM1L/// CGI-04 | Protéine CGI-04 analogue à la dynamine 1 |
| 6 | 53720_at | Hs.175120 | FLJ11286 | Protéine hypothétique FLJ11286 |
| 7 | 202509_s_at | Hs.525607 | TNFAIP2 | Facteur de nécrose tumorale, protéine 2 à induction alpha |
| 8 | 241408_at | Hs.26410 | FLJ34443 | Protéine hypothétique FLJ34443 |
| 9 | 200732_s_at | Hs.227777 | PTP4A1 | Protéine tyrosine phosphatase de type IVA, élément 1 |
| 10 | 228329_at | Hs.4204 | --- | CDNA FLJ30779 fis, clone FEBRA2000815 |
| 11 | 225028_at | Hs.355559 | LOC550643 | Protéine hypothétique LOC550643 |
| 12 | 214440_at | Hs.155956 | NAT1 | N-acétyltransférase 1 (arylamine N-acétyltransférase) |
| 13 | 203405_at | Hs.473838 | DSCR2 | Gène 2 bas de la région critique de syndrome |
| 14 | 213379_at | Hs.144304 | COQ2 | Homologue de coenzyme Q2, prényltransférase (levure) |
| 15 | 214370-at | Hs.416073 | S100A8 | Protéine A8 de fixation de calcium S100 (calgranuline A) |
| 16 | 221190_s_at | Hs.529006 | C18orf8 | Cadre de lecture ouvert 8 du chromosome 18 |
| 17 | 210357_s_at | Hs.433337 | SMOX | Spermine oxydase |
| 18 | 32209_at | Hs.25723 | MTVR1 | Tumeur mammaire de la souris, homologue 1 de récepteur de virus |
